# EUROPEAN PATENT SPECIFICATION

(11) **EP 4 446 429 B1**
(45) Date of publication and mention of the grant of the patent: **22.07.2026**
(21) Application number: 22904121.5
(22) Date of filing: 01.12.2022
(51) Int. Cl.: C12Q 1/6827, C12Q 1/6832, C12Q 1/6806

(54) **AGENT FOR RAISING MELTING TEMPERATURE OF DOUBLE-STRANDED NUCLEIC ACID, AND USES THEREFOR**
MITTEL ZUR ERHÖHUNG DER SCHMELZTEMPERATUR VON DOPPELSTRÄNGIGER NUKLEINSÄURE UND VERWENDUNGEN DAFÜR
AGENT POUR AUGMENTER LA TEMPÉRATURE DE FUSION D'ACIDE NUCLÉIQUE DOUBLE BRIN, ET SES UTILISATIONS

(30) Priority: 10.12.2021 JP 2021201162
(43) Date of publication of application: 16.10.2024
(73) Proprietor: Nitto Boseki Co., Ltd., Fukushima-shi, Fukushima 960-8161 (JP)
(72) Inventor: FUJIMURA Nahohiro, Kawasaki-shi, Kanagawa 210-0821 (JP); TACHIBANA Ami, Kawasaki-shi, Kanagawa 210-0821 (JP); UCHIKI Tomoaki, Kawasaki-shi, Kanagawa 210-0821 (JP); TAKEUCHI Minoru, Koriyama-shi, Fukushima 963-8061 (JP); TERUUCHI Yoko, Koriyama-shi, Fukushima 963-8061 (JP); WATANABE Koji, Koriyama-shi, Fukushima 963-8061 (JP)
(74) Representative: Hoffmann Eitle
(86) International application number: PCT/JP2022/044338
(87) International publication number: WO 2023/106190

(56) References cited:
- EP-A1- 2 554 680
- WO-A1-03/018841
- WO-A1-2023/106189
- JP-A- 2004 537 263
- JP-A- 2008 278 779
- JP-A- 2018 104 850
- JP-A- 2018 104 850
- WANG BAOZHEN ET AL: "Use of Amphoteric Copolymer Films as Sacrificial Layers for Constructing Free-Standing Layer-by-Layer Films", MATER, vol. 6, no. 6, 6 June 2013 (2013-06-06), pages 2351 - 2359, XP093250116, ISSN: 1996-1944, DOI: 10.3390/ma6062351
- FARIN B. ET AL: "NiMoO4 preparation from polyampholytic hybrid precursors: Benefiting of the memory effect in the oxidative dehydrogenation of propane", CATALYSIS TODAY, vol. 203, 1 March 2013 (2013-03-01), AMSTERDAM, NL, pages 24 - 31, XP093250127, ISSN: 0920-5861, DOI: 10.1016/j.cattod.2012.03.070
- RULLENS FRANÇOIS ET AL: "New Regular, Amphiphilic Poly(ampholyte)s: Synthesis and Characterization", MACROMOLECULAR CHEMISTRY AND PHYSICS, vol. 205, no. 9, 28 May 2004 (2004-05-28), DE, pages 1155 - 1166, XP093250130, ISSN: 1022-1352, DOI: 10.1002/macp.200400081
- JIALUN HAN ET AL: "Fluorescent DNA Biosensor for Single-Base Mismatch Detection Assisted by Cationic Comb-Type Copolymer", MOLECULES, vol. 24, no. 3, 5 February 2019 (2019-02-05), DE, pages 575, XP055556584, ISSN: 1433-1373, DOI: 10.3390/molecules24030575
- ROBERTS MARY F: "Organic compatible solutes of halotolerant and halophilic microorganisms", SALINE SYSTEMS, BIOMED CENTRAL, LONDON, GB, vol. 1, no. 1, 4 August 2005 (2005-08-04), pages 5, XP021011381, ISSN: 1746-1448, DOI: 10.1186/1746-1448-1-5
- TACHIBANA AMI ET AL: "Cationic copolymers that enhance wild-type-specific suppression in BNA-clamp PCR and preferentially increase the T m of fully matched complementary DNA and BNA strands", vol. 7, no. 1, 10 January 2022 (2022-01-10), XP093068867, Retrieved from the Internet <URL:https://academic.oup.com/biomethods/article-pdf/7/1/bpac009/43858911/bpac009.pdf> DOI: 10.1093/biomethods/bpac009
- JIALUN HAN, WU JINCAI, DU JIE: "Fluorescent DNA Biosensor for Single-Base Mismatch Detection Assisted by Cationic Comb-Type Copolymer", MOLECULES, SPRINGER VERLAG, BERLIN, DE, vol. 24, no. 3, DE , pages 575, XP055556584, ISSN: 1433-1373, DOI: 10.3390/molecules24030575
- SAKAKI S, NAKABAYASHI N: "MPC polymer ni yoru 1 enki takata (SNPs) no kokateki na ninshiki / EFFECTIVE RECOGNITION OF SINGLE NUCLEOTIDE POLYMORPHISM (SNPs) BY USE OF MPC POLYMERS", POLYMER PREPRINTS. JAPAN, SOCIETY OF POLYMER SCIENCE., JP, vol. 50, no. 5, 1 May 2001 (2001-05-01), JP , pages 992, XP002960296
- TACHIBANA AMI, FUJIMURA NAHOHIRO, TAKEUCHI MINORU, WATANABE KOJI, TERUUCHI YOKO, UCHIKI TOMOAKI: "Cationic copolymers that enhance wild-type-specific suppression in BNA-clamp PCR and preferentially increase the T m of fully matched complementary DNA and BNA strands", BIOLOGY METHODS AND PROTOCOLS, vol. 7, no. 1, 10 January 2022 (2022-01-10), XP093068867, DOI: 10.1093/biomethods/bpac009

## Description

### TECHNICAL FIELD

The invention is set out in the appended set of claims.

### BACKGROUND ART

The detection of mutations in a genomic nucleic acid has been widely performed to identify specific bacteria or viruses or to determine disease susceptibility, drug response in humans, and recently, it has been required to detect mutation at one base in a genomic nucleic acid accurately, rapidly, and at low cost.

Various methods for detecting mutation in a genomic nucleic acid have been developed, and for example, a method for detecting mismatching with respect to a standard nucleic acid in a sample nucleic acid has been proposed, wherein the method comprises allowing a double-stranded nucleic acid consisting of a standard nucleic acid and a complementary strand thereof to coexist with a sample nucleic acid, and measuring the speed or rate at which the complementary strand is replaced with the sample nucleic acid (Patent Literatures 1 to 3). In this method, a polymer obtained by graft-polymerizing a cationic polymer such as polylysine or polyarginine as the main chain, and dextran, polyethylene glycol, or the like as the side chain, is added to promote the rate at which a complementary strand is replaced with a sample nucleic acid, thereby enhancing the detection sensitivity It is thus stated that mismatching even at one-base level can be detected. However, this method requires fluorescence resonance energy transfer (FRET) for detection of the replacement of a complementary strand with a sample nucleic acid, and thus requires labeling the standard nucleic acid and the complementary strand with a fluorescent dye. If this is not necessary, it is more convenient.

As a method that can detect mutation in a genomic nucleic acid without a fluorescent dye-labeled nucleic acid, e.g. for FRET, there are methods of detecting mismatching in a double-stranded nucleic acid with a melting temperature (Tm).

The melting temperature (Tm) means a temperature at which 50% of a double-stranded nucleic acid denatures into single strands, and the melting temperature (Tm) is lower in a mismatch double strand having any un-complementary relation as compared with a full-match double strand having complementary relation. Accordingly, mutation in a sample nucleic acid with respect to a standard nucleic acid can be detected by utilizing this phenomenon, or a target sequence can be detected through a difference in nucleic acid amplification efficiency caused by this phenomenon. However, a change in a melting temperature (Tm) caused by mismatching at one base is considered about 1 to 3°C. Therefore, in order to use a melting temperature (Tm) to detect very few mutations in a genomic nucleic acid, it is necessary to improve the sensitivity.

In this regard, methods that analyze a melting curve to make slight mutation detectable have been developed (Patent Literatures 4 and 5). However, these methods require strict temperature control and thus require high-precision thermostatic devices. Therefore, a simpler method is desired for practical use.

Patent Literature 1 described above also describe that Tm values of a 20-mer double-stranded nucleic acid having mismatching at one base and a full-match 20-mer double-stranded nucleic acid were measured in the presence and absence of the above-mentioned cationic polymers, and shows that a Tm rise value due to the presence of each cationic polymer. Unfortunately, the Tm rise values of the mismatch and full-match double-stranded nucleic acids due to the presence of the cationic polymer are both about 15°C, and there is no significant difference between them.

Patent Literature 3 discloses a solution for use in hybridization of a nucleic acid which comprises a nucleic acid melting temperature adjusting agent and a cationic polymer in the solution.

### CITATION LIST

### PATENT LITERATURE

PATENT LITERATURE 1: WO 03/018841 A1
PATENT LITERATURE 2: JP-A-2001-78769
PATENT LITERATURE 3: JP-A-2008-278779
PATENT LITERATURE 4: JP-A-2005-58107
PATENT LITERATURE 5: JP-A-2003-528626
PATENT LITERATURE 6: WO 2016/167320 A1
PATENT LITERATURE 7: JP-B-6242336
PATENT LITERATURE 8: JP-B-5813263
PATENT LITERATURE 9: JP-B-4731324
PATENT LITERATURE 10: JP-B-4383178
PATENT LITERATURE 11: JP-B-5030998
PATENT LITERATURE 12: JP-B-4151751
PATENT LITERATURE 13: JP-A-2001-89496
PATENT LITERATURE 14: WO 2003/068795
PATENT LITERATURE 15: WO 2005/021570
PATENT LITERATURE 16: JP-B-3756313

### SUMMARY OF INVENTION

### TECHNICAL PROBLEM

An object of the present disclosure is to provide a novel agent for raising a melting temperature (Tm) of a double-stranded nucleic acid, a method for detecting mismatching in a double-stranded nucleic acid or mutation in a sample nucleic acid with respect to a standard nucleic acid, and a kit for carrying out the method, which can address the problems in the prior art as described above.

### SOLUTION TO PROBLEM

As a result of intensive studies, the present inventors have found that when an amphoteric copolymer comprising a specific cationic constituent unit (1) and a specific anionic constituent unit (2) (hereinafter, may also be abbreviated as "specific amphoteric copolymer") is added to a nucleic acid solution, the presence of the specific amphoteric copolymer increases a difference between a Tm rise value of a full-match double-stranded nucleic acid having a complementary relation and a Tm rise value of a mismatch double-stranded nucleic acid having an un-complementary relation, which enables detection of mismatching even at one-base level.

That is, the present invention provides the use of an agent for raising a melting temperature of a double-stranded nucleic acid, comprising an amphoteric copolymer, wherein the amphoteric copolymer comprises:
at least one type of cationic constituent unit (1) selected from the group consisting of
a constituent unit (1-1) having a structure represented by general formula (I-a) or general formula (I-b), or a structure of an acid addition salt thereof: 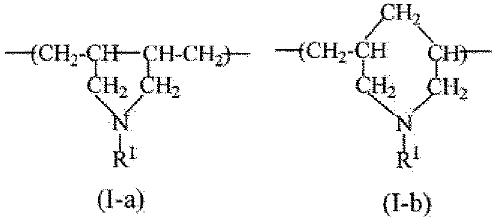
wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,
   and
a constituent unit (1-4) having a structure represented by general formula (1-f), or a structure of an acid addition salt thereof: 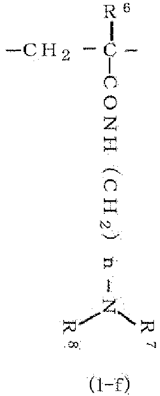
wherein R⁶ represents a hydrogen atom or a methyl group, R⁷ and R⁸ each independently represent an alkyl group having 1 to 4 carbon atoms, and n is an integer of 2 to 4; and
at least one type of anionic constituent unit (2) selected from the group consisting of
a constituent unit (2-1) having a structure represented by general formula (II-a): 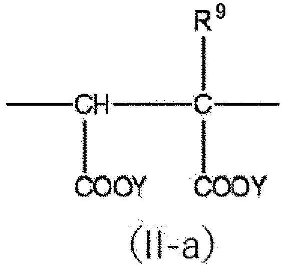
wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded,
   and
a constituent unit (2-4) having a structure represented by general formula (II-d) 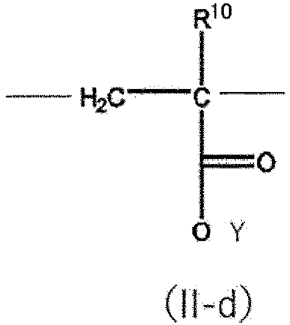
wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

The present invention also provides a method for detecting nucleic acid mismatching between a sample nucleic acid and a probe nucleic acid, comprising the steps of:
(1) measuring melting temperatures of a double-stranded nucleic acid between the probe nucleic acid and a nucleic acid having a base sequence complementary to the probe nucleic acid with and without adding an agent for raising a melting temperature of a double-stranded nucleic acid, and determining a rise value between the melting temperatures of the probe nucleic acid and the nucleic acid having a base sequence complementary to the probe nucleic acid due to the presence of the amphoteric copolymer (standard ΔTm);
(2) measuring melting temperatures of a double-stranded nucleic acid between the sample nucleic acid and the probe nucleic acid with and without adding an agent for raising a melting temperature of a double-stranded nucleic acid, and determining a rise value between the melting temperatures of the double-stranded nucleic acid of the sample nucleic acid and the probe nucleic acid due to the presence of the amphoteric copolymer (sample ΔTm); and
(3) determining the presence or absence of nucleic acid mismatching based on a value (ΔΔTm) obtained by subtracting the sample ΔTm from the standard ΔTm, wherein the agent for raising a melting temperature of a double-stranded nucleic acid comprises:
   an amphoteric copolymer, wherein the amphoteric copolymer comprises:
   at least one kind of cationic constituent unit (1) selected from the group consisting of
   a constituent unit (1-1) having a structure represented by general formula (I-a) or general formula (I-b), or a structure of an acid addition salt thereof: 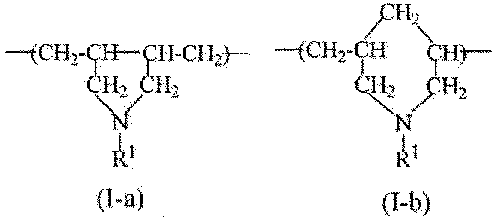
   wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
   a constituent unit (1-4) having a structure represented by general formula (1-f), or a structure of an acid addition salt thereof: 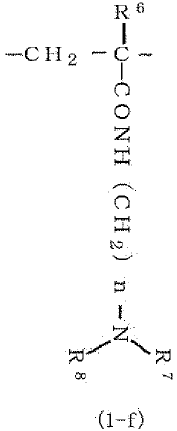
   wherein R⁶ represents a hydrogen atom or a methyl group, R⁷ and R⁸ each independently represent an alkyl group having 1 to 4 carbon atoms, and n is an integer of 2 to 4; and
   at least one kind of anionic constituent unit (2) selected from the group consisting of
   a constituent unit (2-1) having a structure represented by general formula (II-a): 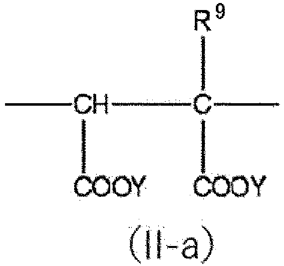
   wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
   a constituent unit (2-4) having a structure represented by general formula (II-d): 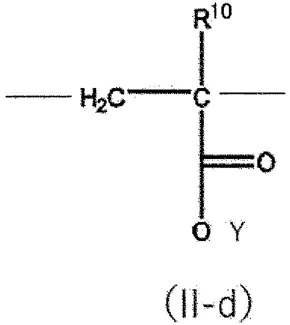
   wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

The present invention also provides a kit for detecting nucleic acid mismatching between a sample nucleic acid and a probe nucleic acid, comprising:
the probe nucleic acid;
a nucleic acid having a base sequence complementary to the probe nucleic acid; and
an agent for raising a melting temperature of a double-stranded nucleic acid wherein the agent for raising a melting temperature of a double-stranded nucleic acid comprises:
   an amphoteric copolymer, wherein the amphoteric copolymer comprises:
   at least one kind of cationic constituent unit (1) selected from the group consisting of
   a constituent unit (1-1) having a structure represented by general formula (I-a) or general formula (I-b), or a structure of an acid addition salt thereof: 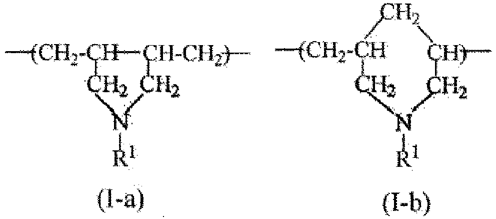
   wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and a constituent unit (1-4) having a structure represented by general formula (1-f), or a structure of an acid addition salt thereof: 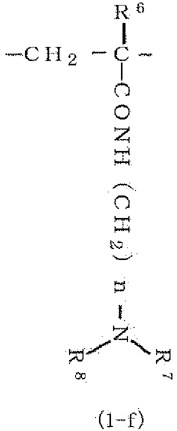
   wherein R⁶ represents a hydrogen atom or a methyl group, R⁷ and R⁸ each independently represent an alkyl group having 1 to 4 carbon atoms, and n is an integer of 2 to 4; and
   at least one kind of anionic constituent unit (2) selected from the group consisting of
   a constituent unit (2-1) having a structure represented by general formula (II-a): 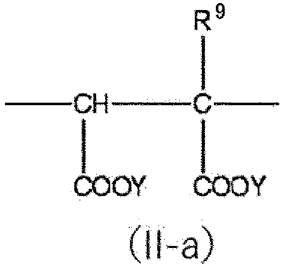
   wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
   a constituent unit (2-4) having a structure represented by general formula (II-d): 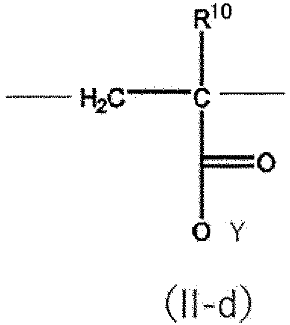
   wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

The present invention also provides a method for detecting mutation with respect to a standard nucleic acid in a sample nucleic acid, comprising the steps of:
(1) measuring melting temperatures of a double-stranded nucleic acid between the standard nucleic acid and a nucleic acid having a base sequence complementary to the standard nucleic acid with and without adding an agent for raising a melting temperature of a double-stranded nucleic acid, and determining a rise value (standard ΔTm) between the melting temperatures of the double-stranded nucleic acid between the standard nucleic acid and the nucleic acid having the base sequence complementary to the standard nucleic acid due to the presence of the amphoteric copolymer;
(2) measuring melting temperatures of a double-stranded nucleic acid of the sample nucleic acid and the nucleic acid having a base sequence complementary to the standard nucleic acid with and without adding an agent for raising a melting temperature of a double-stranded nucleic acid, and determining a rise value (sample ΔTm) between the melting temperatures of the double-stranded nucleic acid between the sample nucleic acid and the nucleic acid having the base sequence complementary to the standard nucleic acid due to the presence of the amphoteric copolymer; and
(3) determining the presence or absence of mutation based on a value (ΔΔTm) obtained by subtracting the sample ΔTm from the standard ΔTm,
   wherein the agent for raising a melting temperature of a double-stranded nucleic acid comprises:
      an amphoteric copolymer, wherein the amphoteric copolymer comprises:
      at least one kind of cationic constituent unit (1) selected from the group consisting of
      a constituent unit (1-1) having a structure represented by general formula (I-a) or general formula (I-b), or a structure of an acid addition salt thereof: 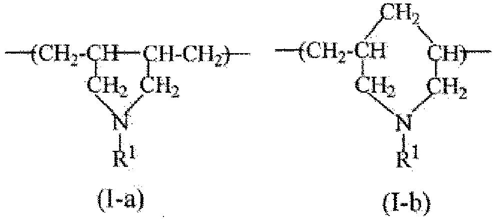
      wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
      a constituent unit (1-4) having a structure represented by general formula (1-f), or a structure of an acid addition salt thereof: 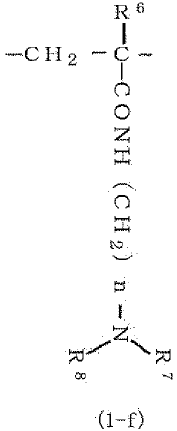
      wherein R⁶ represents a hydrogen atom or a methyl group, R⁷ and R⁸ each independently represent an alkyl group having 1 to 4 carbon atoms, and n is an integer of 2 to 4; and
      at least one kind of anionic constituent unit (2) selected from the group consisting of
      a constituent unit (2-1) having a structure represented by general formula (II-a): 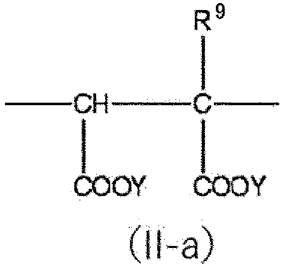
      wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and a constituent unit (2-4) having a structure represented by general formula (II-d): 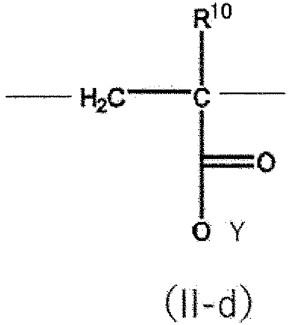
      wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

The present invention also provides a kit for the detecting mutation between a sample nucleic acid and a standard nucleic acid, comprising:
the standard nucleic acid;
a nucleic acid having a base sequence complementary to the standard nucleic acid; and
an agent for raising a melting temperature of a double-stranded nucleic acid,
wherein the agent for raising a melting temperature of a double-stranded nucleic acid comprises:
   an amphoteric copolymer, wherein the amphoteric copolymer comprises:
   at least one kind of cationic constituent unit (1) selected from the group consisting of
   a constituent unit (1-1) having a structure represented by general formula (I-a) or general formula (I-b), or a structure of an acid addition salt thereof: 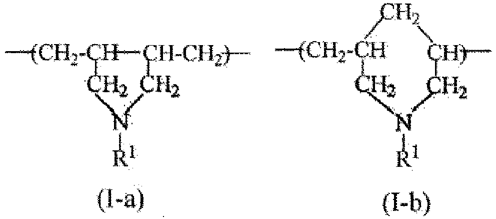
   wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
   a constituent unit (1-4) having a structure represented by general formula (1-f), or a structure of an acid addition salt thereof: 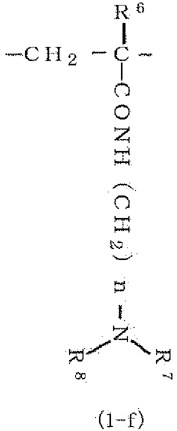
   wherein R⁶ represents a hydrogen atom or a methyl group, R⁷ and R⁸ each independently represent an alkyl group having 1 to 4 carbon atoms, and n is an integer of 2 to 4; and
   at least one kind of anionic constituent unit (2) selected from the group consisting of
   a constituent unit (2-1) having a structure represented by general formula (II-a): 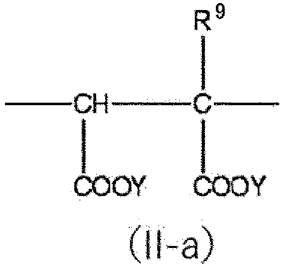
   wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
   a constituent unit (2-4) having a structure represented by general formula (II-d): 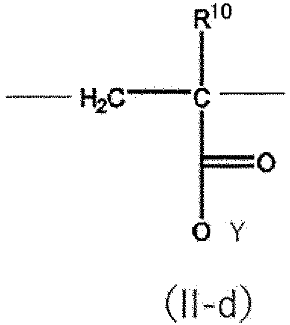
   wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

According to the present disclosure, mismatching of a double-stranded nucleic acid can be detected simply and with high sensitivity by using a melting temperature (Tm) of the double-stranded nucleic acid. Therefore, the presence or absence of mutation in a sample nucleic acid with respect to a standard nucleic acid (for example, wild-type genomic nucleic acid) can be detected simply and with high sensitivity by using a melting temperature (Tm) of a double-stranded nucleic acid.

### BRIEF DESCRIPTION OF DRAWINGS

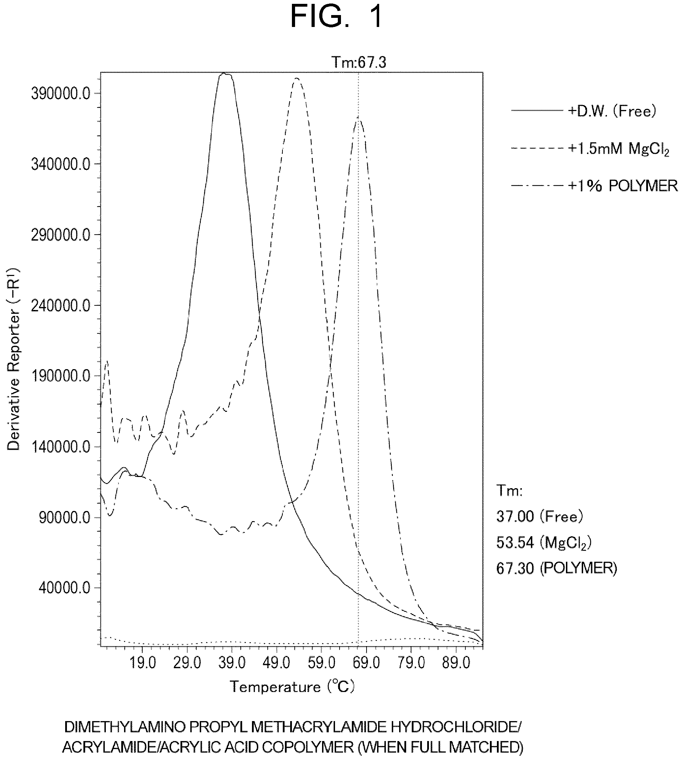

FIG. 1 shows melting curves of a double-stranded between a standard nucleic acid oligomer and a complementary strand oligomer in the presence and absence of a dimethylamino propyl methacrylamide hydrochloride/acrylamide/acrylic acid copolymer, and in the presence of MgCl₂ in place of the copolymer.

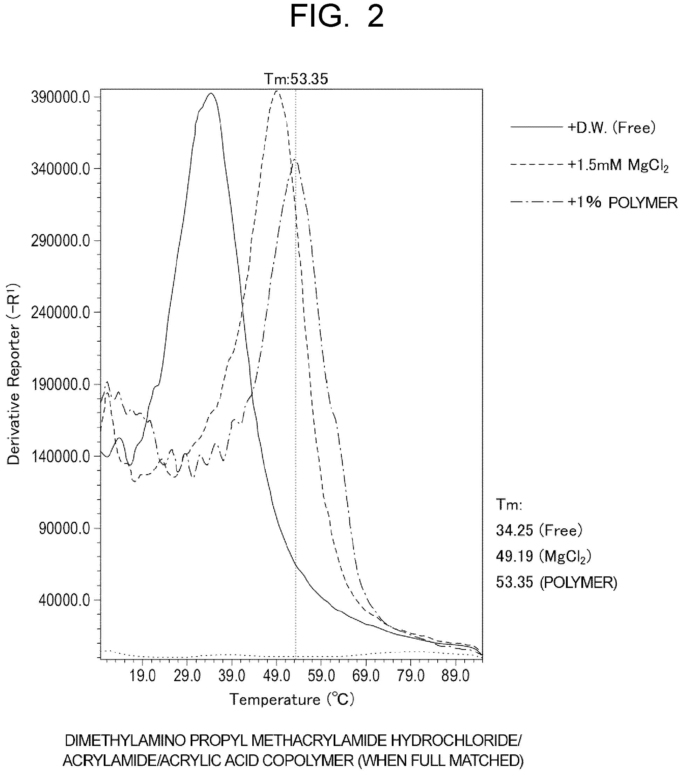

FIG. 2 shows melting curves of a double-stranded between a nucleic acid oligomer having mutation at one base with respect to a standard nucleic acid oligomer and an oligomer strand fully complementary to the standard nucleic acid oligomer in the presence and absence of a dimethylamino propyl methacrylamide hydrochloride/acrylamide/acrylic acid copolymer, and in the presence of MgCl₂ in place of the copolymer.

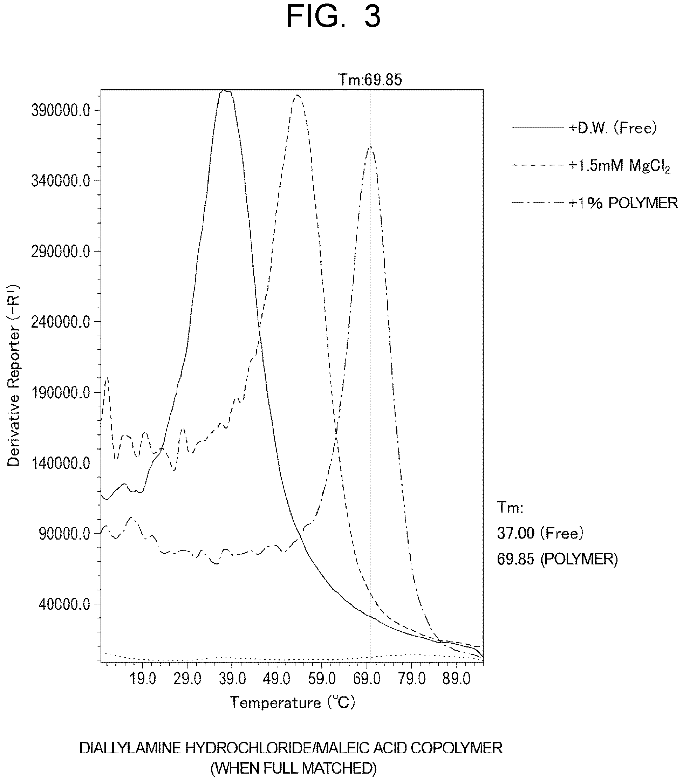

FIG. 3 shows melting curves of a double-stranded between a standard nucleic acid oligomer and a complementary strand oligomer in the presence and absence of a diallylamine hydrochloride/maleic acid copolymer, and in the presence of MgCl₂ in place of the copolymer.

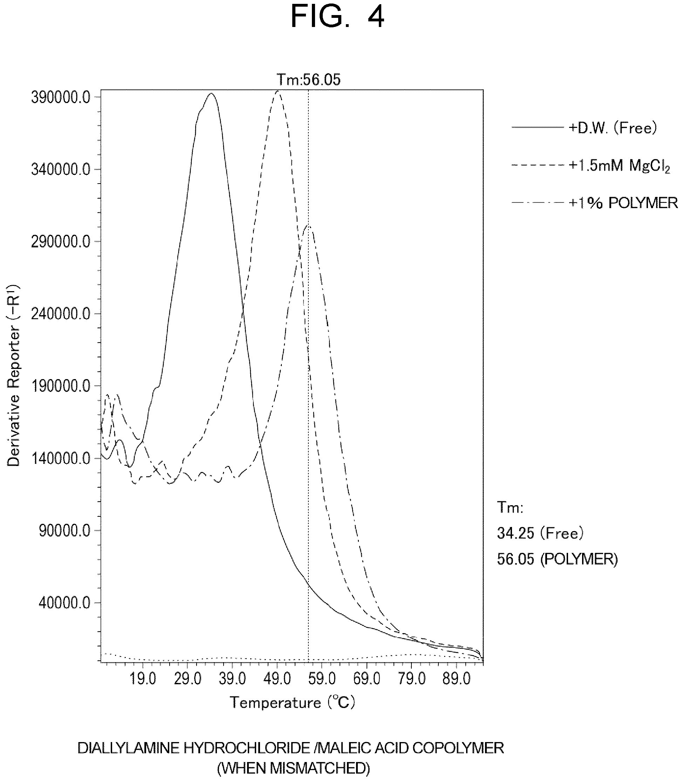

FIG. 4 shows melting curves of a double-stranded between a nucleic acid oligomer having mutation at one base with respect to a standard nucleic acid oligomer and an oligomer strand fully complementary to the standard nucleic acid oligomer in the presence and absence of a diallylamine hydrochloride/maleic acid copolymer, and in the presence of MgCl₂ in place of the copolymer.

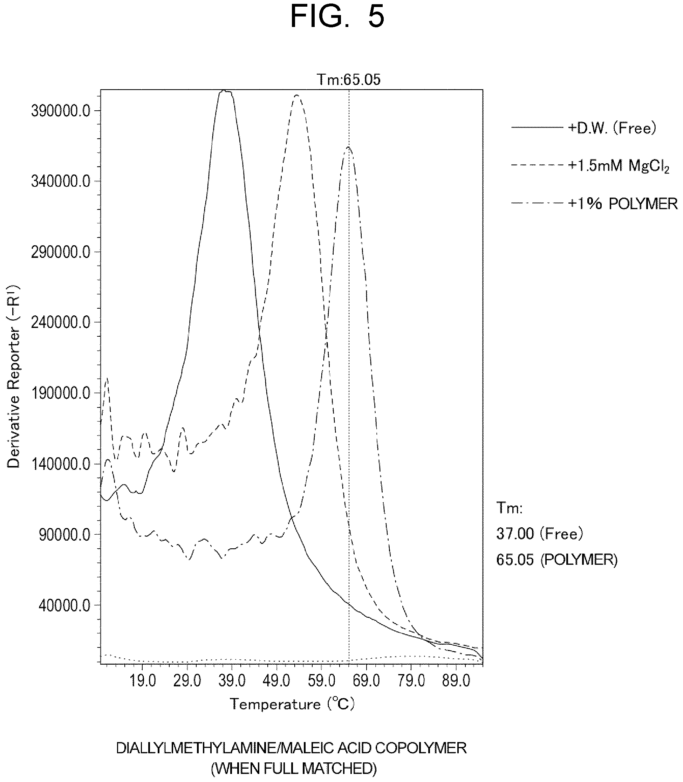

FIG. 5 shows melting curves of a double-stranded between a standard nucleic acid oligomer and a complementary strand oligomer in the presence and absence of a diallylmethylamine/maleic acid copolymer, and in the presence of MgCl₂ in place of the copolymer.

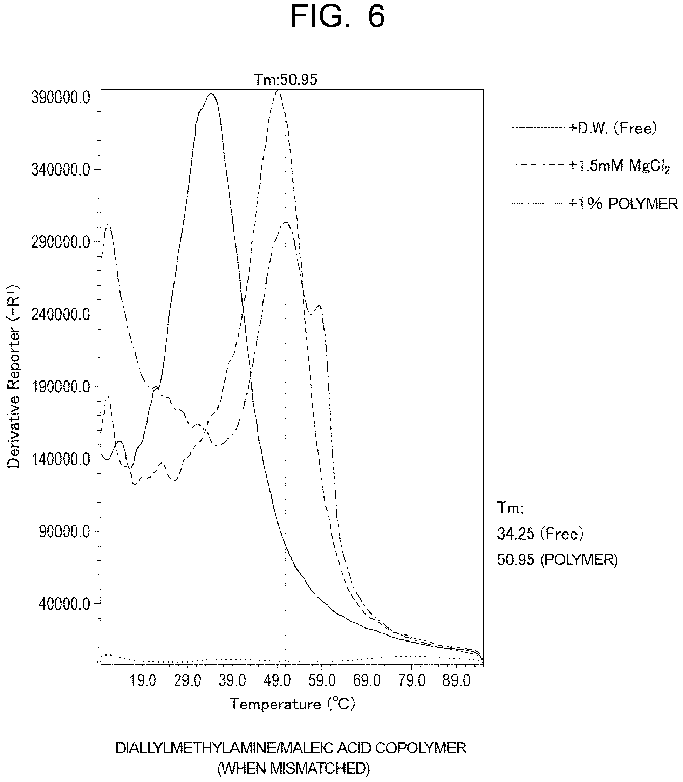

FIG. 6 shows melting curves of a double-stranded between a nucleic acid oligomer having mutation at one base with respect to a standard nucleic acid oligomer and an oligomer strand fully complementary to the standard nucleic acid oligomer in the presence and absence of a diallylmethylamine/maleic acid copolymer, and in the presence of MgCl₂ in place of the copolymer.

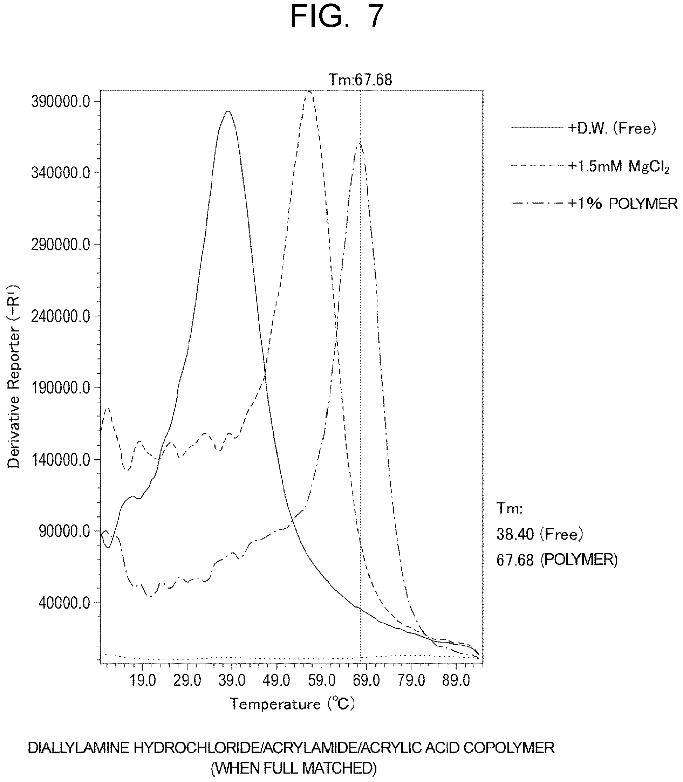

FIG. 7 shows melting curves of a double-stranded between a standard nucleic acid oligomer and a complementary strand oligomer in the presence or absence of a diallylamine hydrochloride/acrylamide/acrylic acid copolymer, and in the presence of MgCl₂ in place of the copolymer.

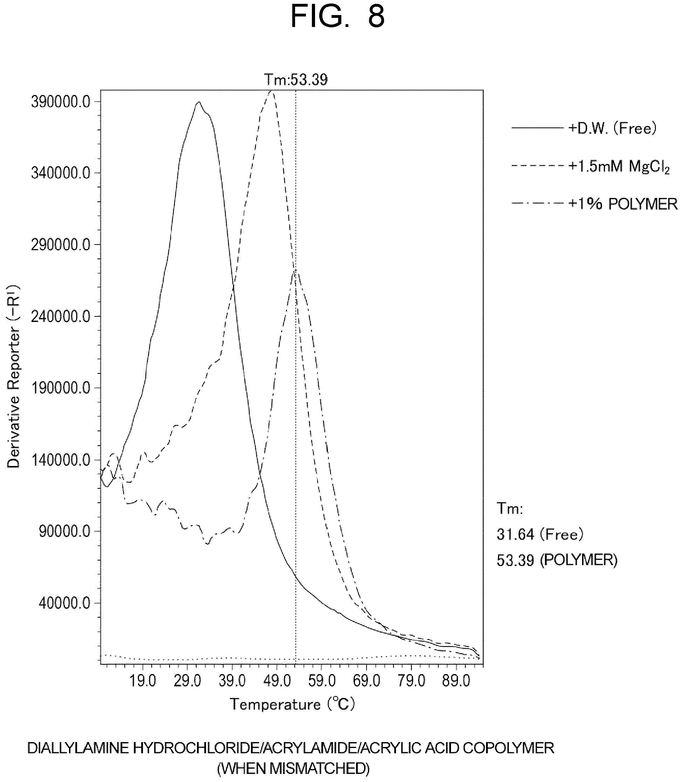

FIG. 8 shows melting curves of a double-stranded between a nucleic acid oligomer having mutation at one base with respect to a standard nucleic acid oligomer and an oligomer strand complementary to the standard nucleic acid oligomer in the presence and absence of a diallylamine hydrochloride/acrylamide/acrylic acid copolymer, and in the presence of MgCl₂ in place of the copolymer.

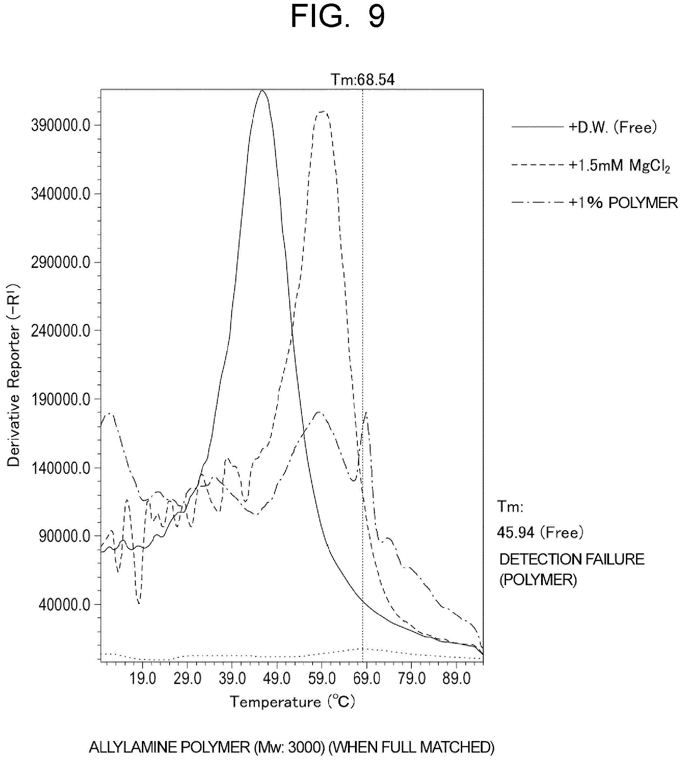

FIG. 9 shows melting curves of a double-stranded between a standard nucleic acid oligomer and a fully complementary strand oligomer in the presence and absence of an allylamine polymer (weight average molecular weight (Mw): 3000), and in the presence of MgCl₂ in place of the copolymer.

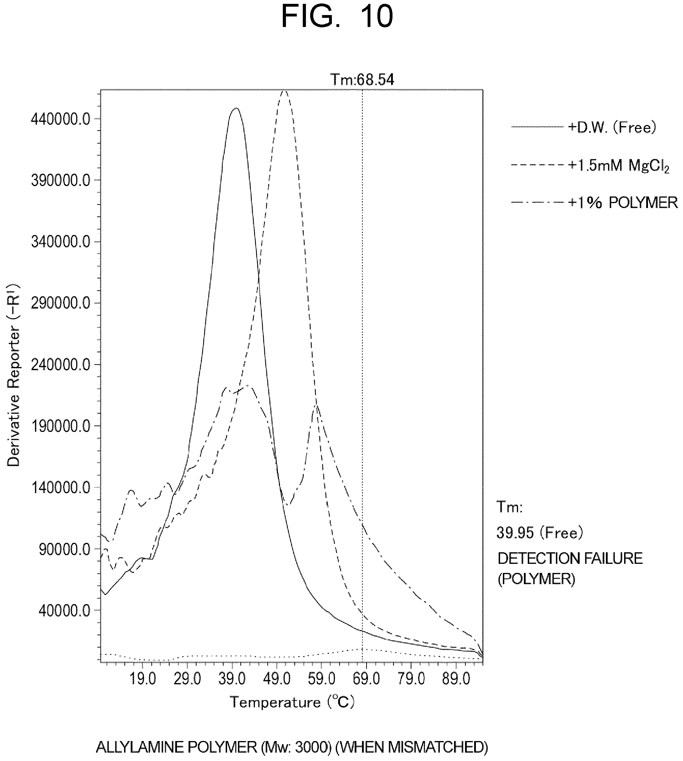

FIG. 10 shows melting curves of a double-stranded between a nucleic acid oligomer having mutation at one base with respect to a standard nucleic acid oligomer and an oligomer strand complementary to the standard nucleic acid oligomer in the presence and absence of an allylamine polymer (weight average molecular weight (Mw): 3000), and in the presence of MgCl₂ in place of the copolymer.

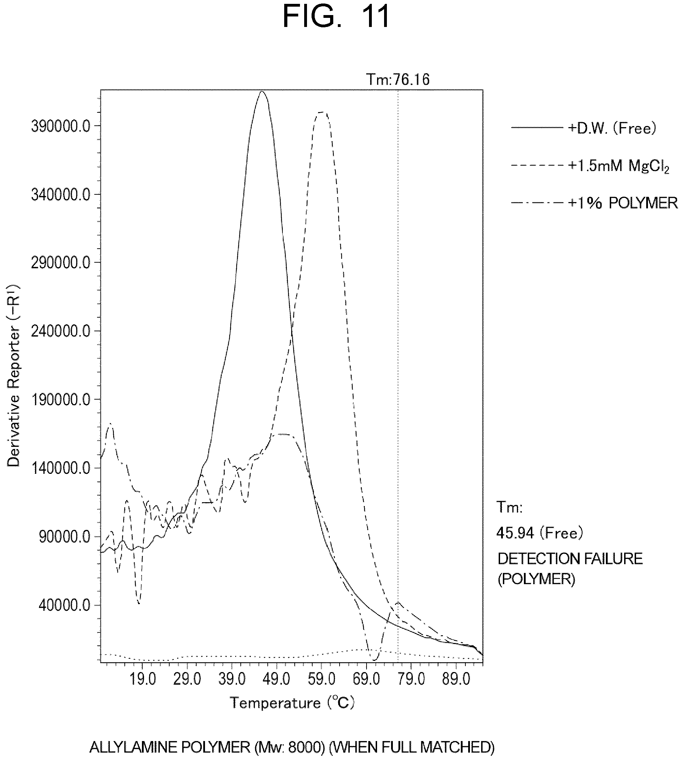

FIG. 11 shows melting curves of a double-stranded between a standard nucleic acid oligomer and a fully complementary strand oligomer in the presence and absence of an allylamine polymer (weight average molecular weight (Mw): 8000), and in the presence of MgCl₂ in place of the copolymer.

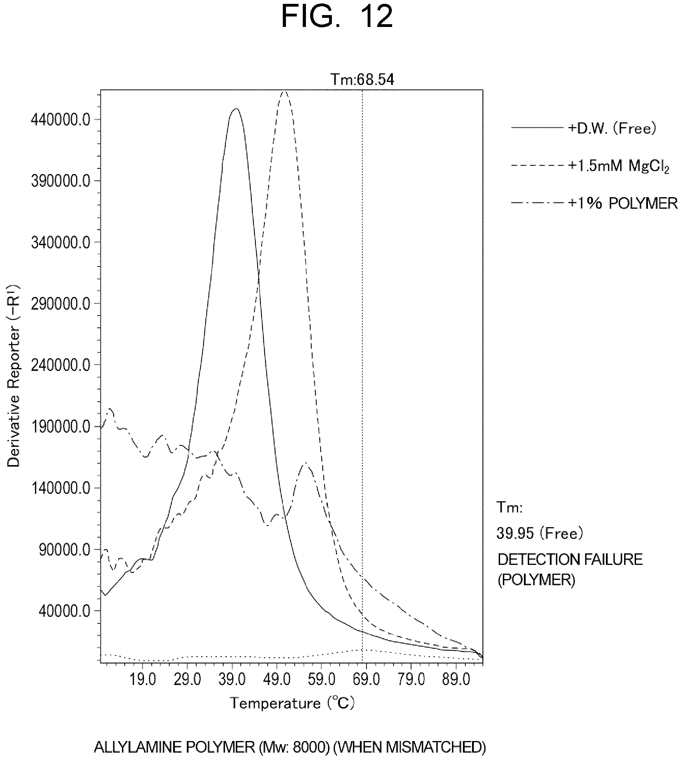

FIG. 12 shows melting curves of a double-stranded between a nucleic acid oligomer having a mutation at one base with respect to a standard nucleic acid oligomer and an oligomer strand complementary to the standard nucleic acid oligomer in the presence and absence of an allylamine polymer (weight average molecular weight (Mw): 8000), and in the presence of MgCl₂ in place of the copolymer.

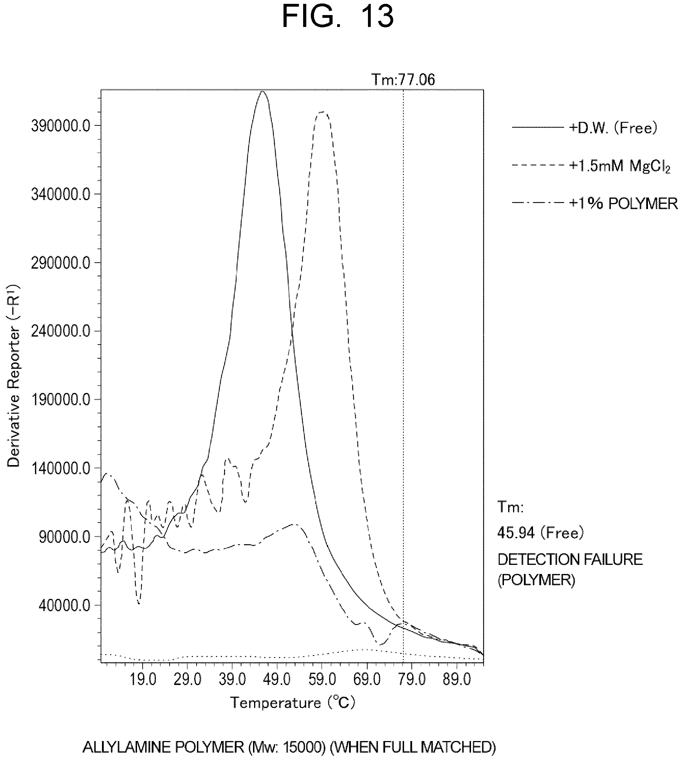

FIG. 13 shows melting curves of a double-stranded between a standard nucleic acid oligomer and a complementary strand oligomer in the presence and absence of an allylamine polymer (weight average molecular weight (Mw): 15000), and in the presence of MgCl₂ in place of the copolymer.

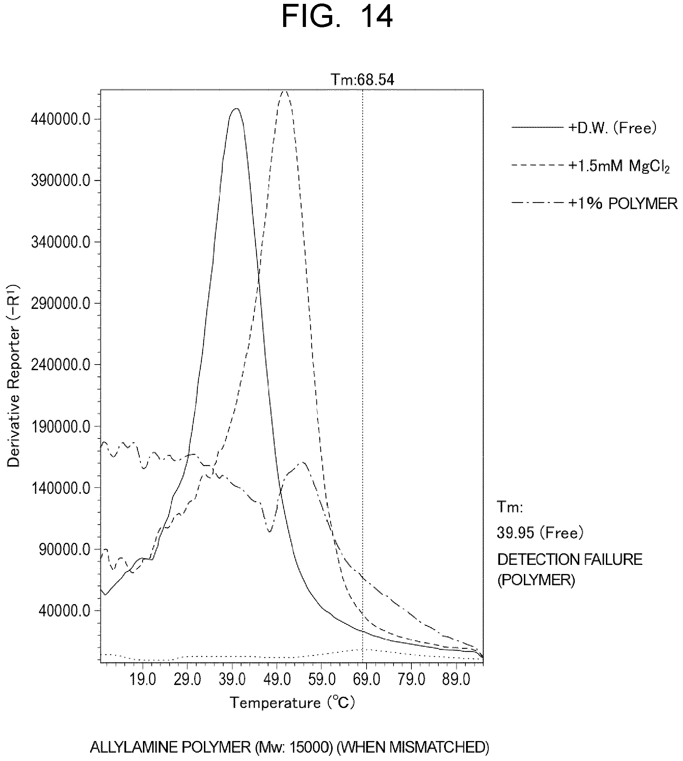

FIG. 14 shows melting curves of a double-stranded between a nucleic acid oligomer having a mutation at one base with respect to a standard nucleic acid oligomer and an oligomer strand fully complementary to the standard nucleic acid oligomer in the presence and absence of an allylamine polymer (weight average molecular weight (Mw): 15000), and in the presence of MgCl₂ in place of the copolymer.

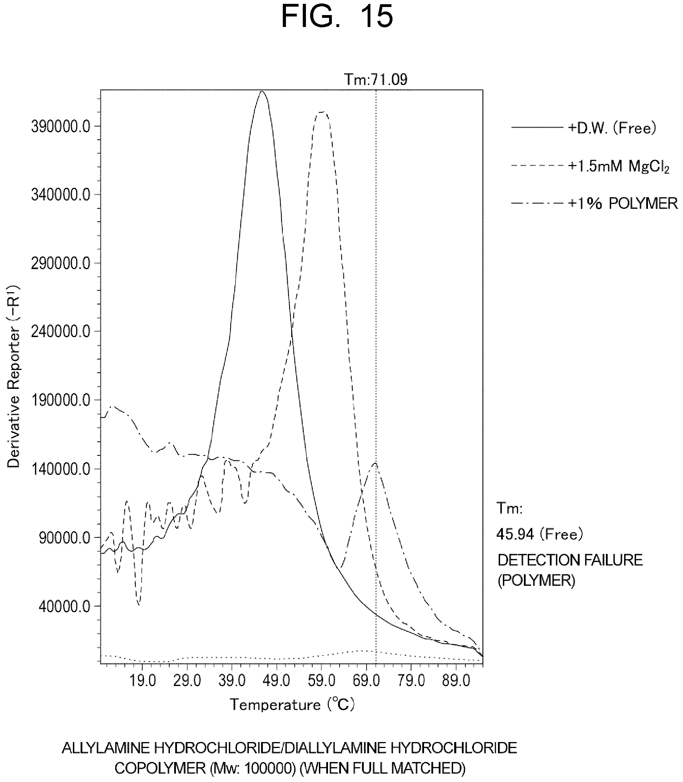

FIG. 15 shows melting curves of a double-stranded between a standard nucleic acid oligomer and a complementary strand oligomer in the presence and absence of an allylamine hydrochloride/diallylamine hydrochloride copolymer (weight average molecular weight (Mw): 100000), and in the presence of MgCl₂ in place of the copolymer.

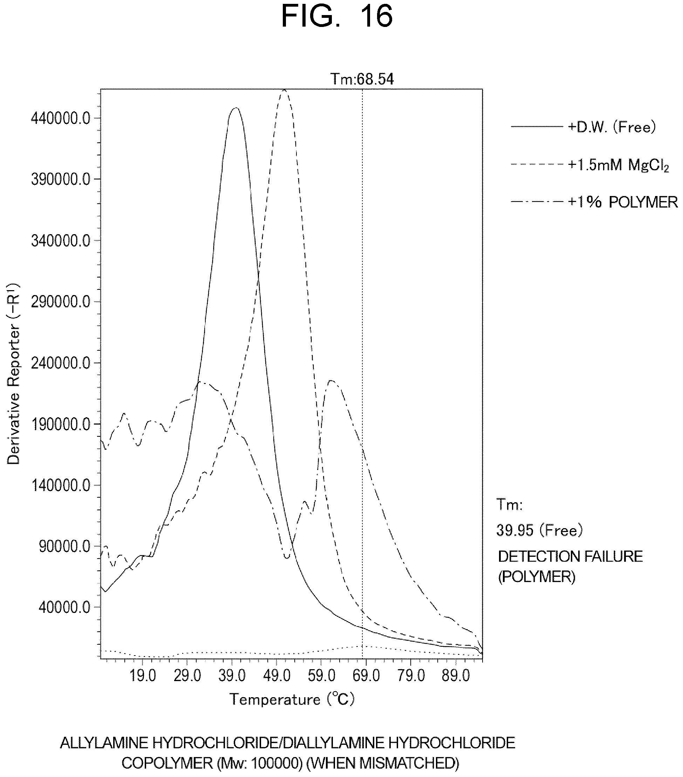

FIG. 16 shows melting curves of a double-stranded between a nucleic acid oligomer having mutation at one base with respect to a standard nucleic acid oligomer and an oligomer strand fully complementary to a standard nucleic acid oligomer in the presence and absence of an allylamine hydrochloride/diallylamine hydrochloride copolymer (weight average molecular weight (Mw): 100000), and in the presence of MgCl₂ in place of the copolymer.

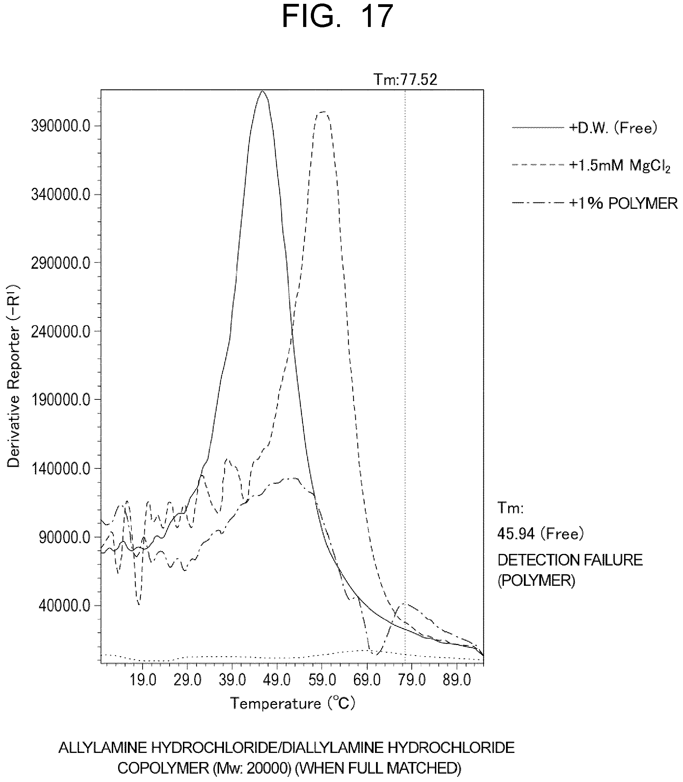

FIG. 17 shows melting curves of a double-stranded between a standard nucleic acid oligomer and a complementary strand oligomer in the presence and absence of an allylamine hydrochloride/diallylamine hydrochloride copolymer (weight average molecular weight (Mw): 20000), and in the presence of MgCl₂ in place of the copolymer.

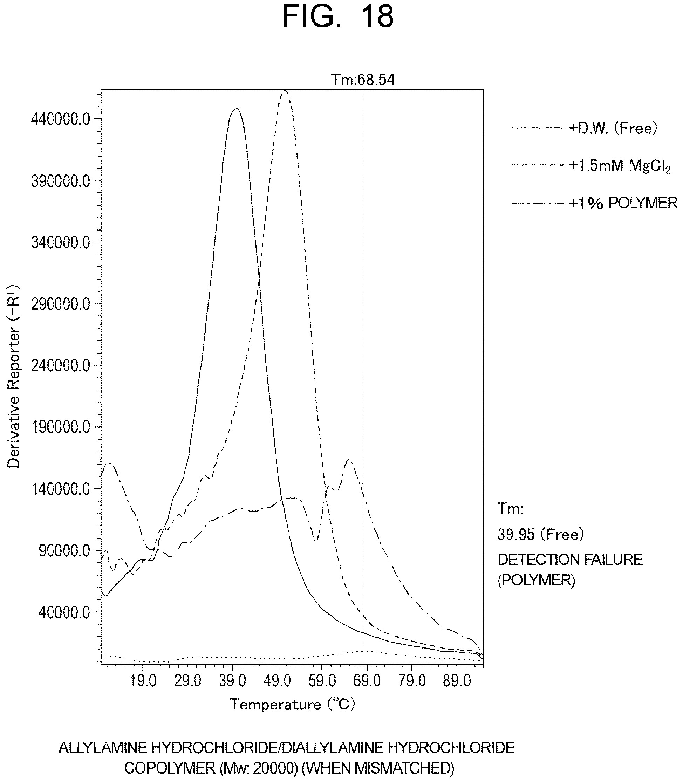

FIG. 18 shows melting curves of a double-stranded between a nucleic acid oligomer having a mutation at one base with respect to a standard nucleic acid oligomer and an oligomer strand fully complementary to a standard nucleic acid oligomer in the presence and absence of an allylamine hydrochloride/diallylamine hydrochloride copolymer (weight average molecular weight (Mw): 20000), and in the presence of MgCl₂ in place of the copolymer.

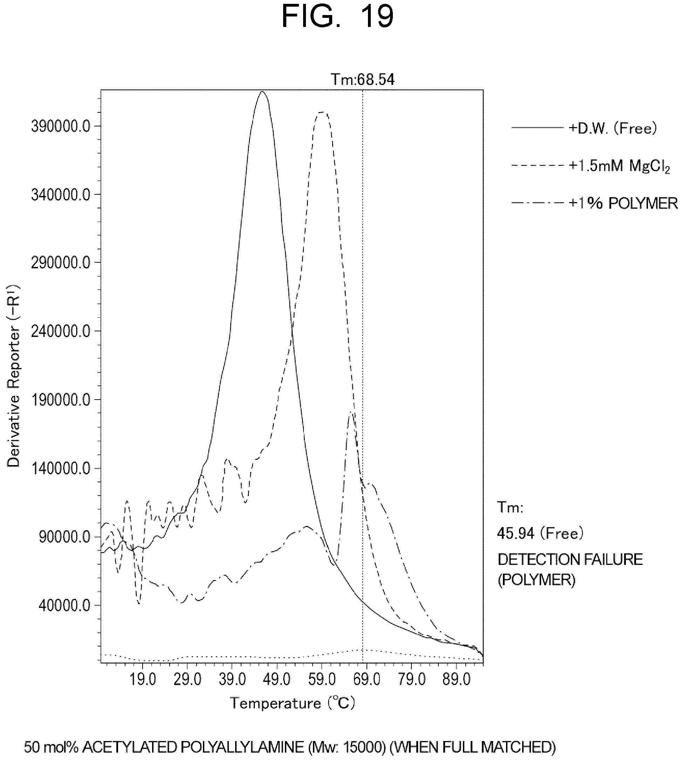

FIG. 19 shows melting curves of a double-stranded between a standard nucleic acid oligomer and a complementary strand oligomer in the presence and absence of 50 mol% acetylated polyallylamine (weight average molecular weight (Mw): 15000), and in the presence of MgCl₂ in place of the copolymer.

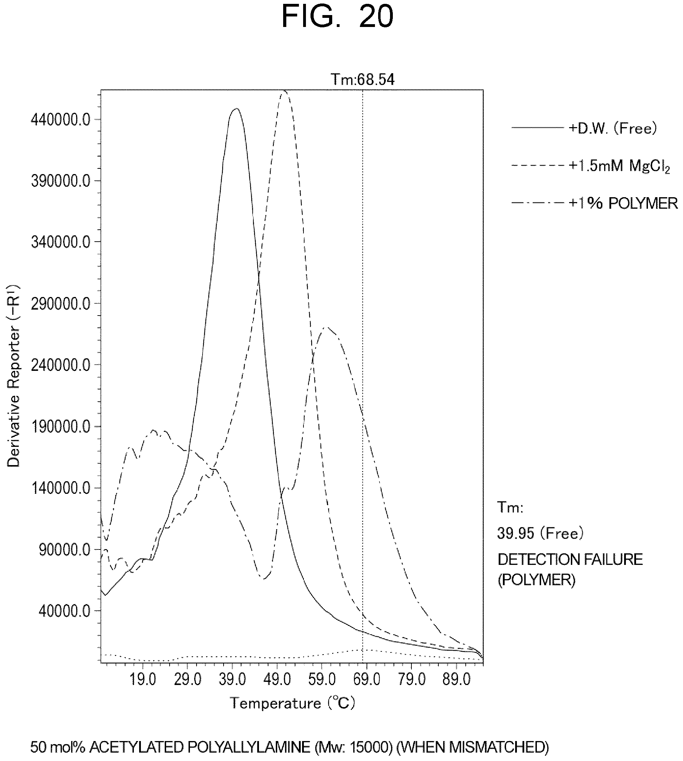

FIG. 20 shows melting curves of a double-stranded between a nucleic acid oligomer having a mutation at one base with respect to a standard nucleic acid oligomer and an oligomer strand fully complementary to the standard nucleic acid oligomer in the presence and absence of 50 mol% acetylated polyallylamine (weight average molecular weight (Mw): 15000), and in the presence of MgCl₂ in place of the copolymer.

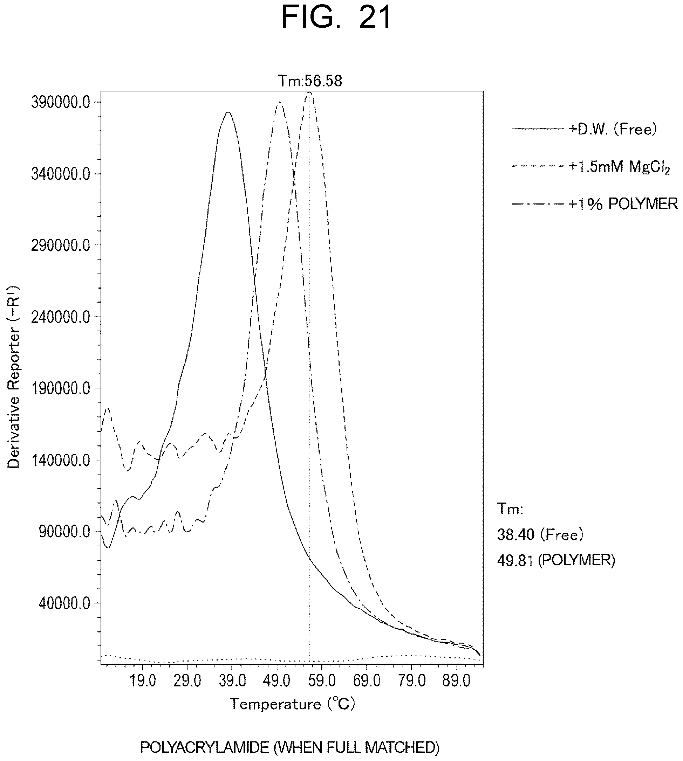

FIG. 21 shows melting curves of a double-stranded between a standard nucleic acid oligomer and a complementary strand oligomer in the presence and absence of polyacrylamide, and in the presence of MgCl₂ in place of the copolymer.

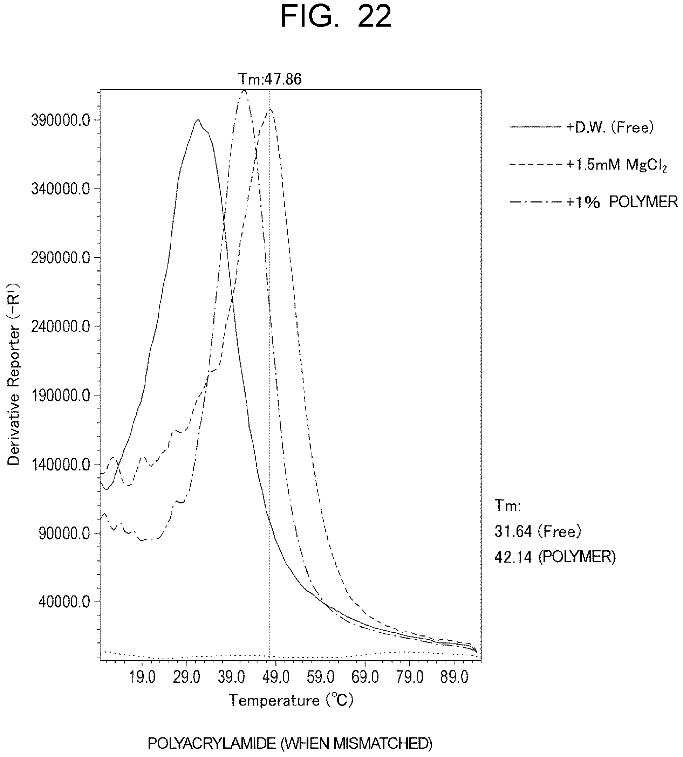

FIG. 22 shows melting curves of a double-stranded between a nucleic acid oligomer having a mutation at one base with respect to a standard nucleic acid oligomer and an oligomer strand fully complementary to the standard nucleic acid oligomer in the presence and absence of polyacrylamide, and in the presence of MgCl₂ in place of the copolymer.

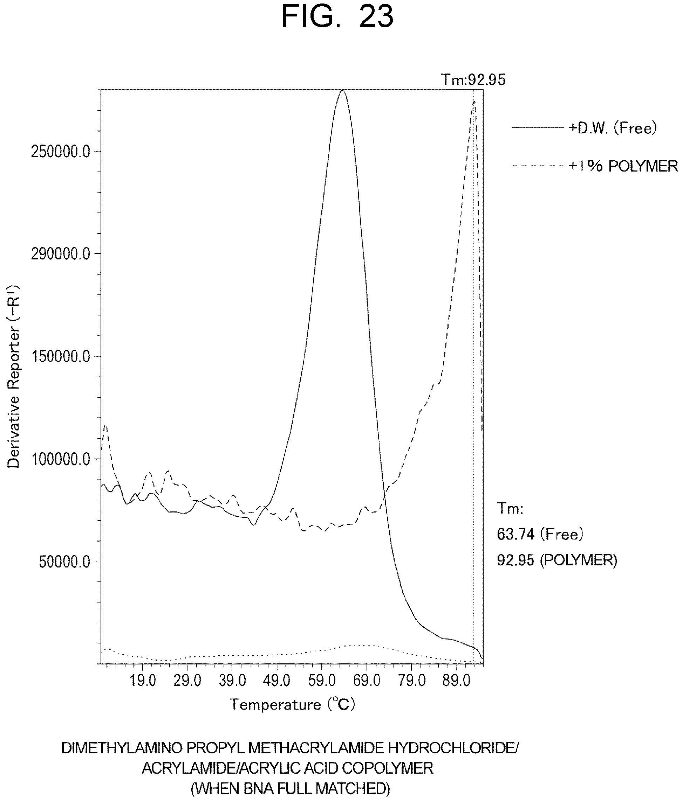

FIG. 23 shows melting curves of a double-stranded between a standard nucleic acid oligomer and a BNA (bridged nucleic acid) complementary strand oligomer in the presence and absence of a dimethylamino propyl methacrylamide hydrochloride/acrylamide/acrylic acid copolymer.

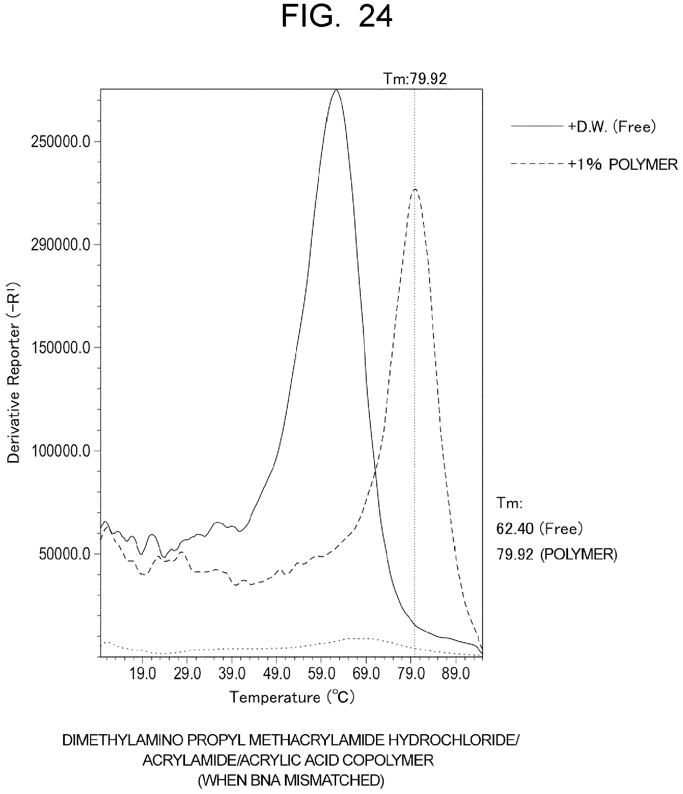

FIG. 24 shows melting curves of a double-stranded between a nucleic acid oligomer having a mutation at one base with respect to a standard nucleic acid oligomer and a BNA oligomer strand complementary to the standard nucleic acid oligomer in the presence and absence of a dimethylamino propyl methacrylamide hydrochloride/acrylamide/acrylic acid copolymer.

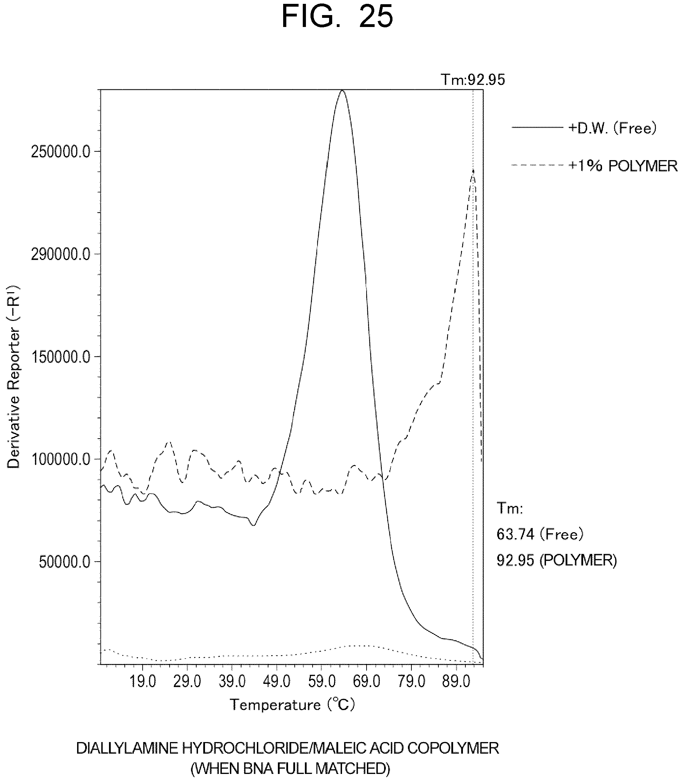

FIG. 25 shows melting curves of a double-stranded between a standard nucleic acid oligomer and a BNA complementary strand oligomer in the presence and absence of a diallylamine hydrochloride/maleic acid copolymer.

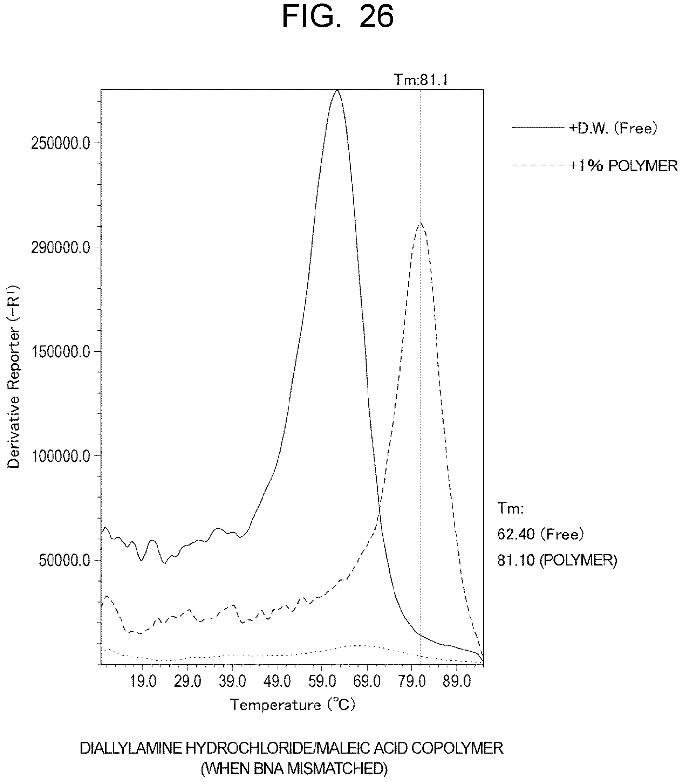

FIG. 26 shows melting curves of a double-stranded melting curve between a nucleic acid oligomer having a mutation at one base with respect to a standard nucleic acid oligomer and a BNA oligomer strand complementary to the standard nucleic acid oligomer in the presence and absence of a diallylamine hydrochloride/maleic acid copolymer.

### DESCRIPTION OF EMBODIMENTS

The invention is set out in the appended set of claims. Embodiments of the present invention will be described in detail.

### 1. Use of an agent for raising melting temperature (Tm) of double-stranded nucleic acid

One embodiment of the present invention relates to the use of an agent for raising a melting temperature of a double-stranded nucleic acid, comprising an amphoteric copolymer, wherein the amphoteric copolymer comprises:
at least one kind of cationic constituent unit (1) selected from the group consisting of
a constituent unit (1-1) having a structure represented by general formula (I-a) or general formula (I-b), or a structure of an acid addition salt thereof: 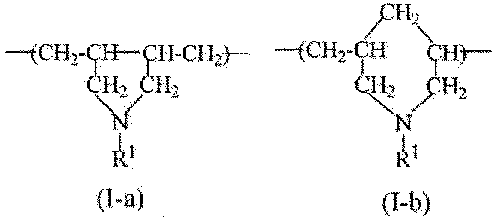
wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
a constituent unit (1-4) having a structure represented by general formula (1-f), or a structure of an acid addition salt thereof: 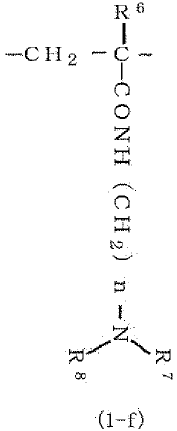
wherein R⁶ represents a hydrogen atom or a methyl group, R⁷ and R⁸ each independently represent an alkyl group having 1 to 4 carbon atoms, and n is an integer of 2 to 4; and
at least one kind of anionic constituent unit (2) selected from the group consisting of
a constituent unit (2-1) having a structure represented by general formula (II-a): 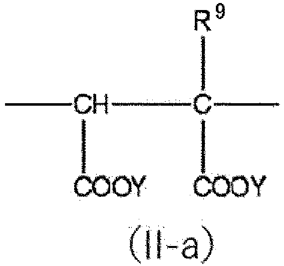
wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
a constituent unit (2-4) having a structure represented by general formula (II-d): 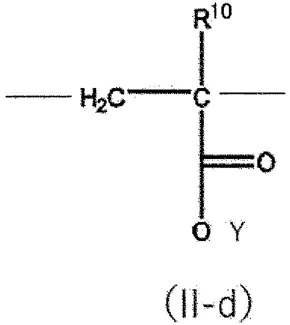
wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded. The agent for raising a Tm of a double-stranded nucleic acid may consist of the specific amphoteric copolymer, described above, or may comprise a component other than the specific amphoteric copolymer, described above, as in the case of an aqueous solution obtained by dissolving the specific amphoteric copolymer, described above, in a nuclease-free medium (for example, water).

The agent for raising a Tm of a double-stranded nucleic acid raises a melting temperature of a double-stranded nucleic acid by 16.0°C or more, and preferably by 20.0°C or more. In particular, in a case where the double-stranded nucleic acid is a double-stranded nucleic acid containing no mismatching (full-match double-stranded nucleic acid), the agent for raising a Tm of a double-stranded nucleic acid raises a melting temperature of the full-match double-stranded nucleic acid by 20.0°C or more, preferably 25.0°C or more, and particularly preferably 30.0°C or more.

### Cationic constituent unit (1)

A cationic constituent unit (1) constituting a specific amphoteric copolymer is at least one type of cationic constituent unit selected from the group consisting of constituent units (1-1) and (1-4).

As shown below using general formulas or the like, the cationic constituent unit (1) selected from the group consisting of constituent units (1-1) and (1-4), has an amino group as the cationic group in the structure, and from the viewpoint of realizing a favorable Tm-value rising effect, and the like, the amino group is a secondary or tertiary amino group.

In the specific amphoteric copolymer, since mismatching in a double-stranded nucleic acid or mutation with respect to a standard nucleic acid in a sample nucleic acid is more reliably detectable, the cationic constituent unit (1) is most preferably the constituent unit (1-1).

### Constituent unit (1-1)

A constituent unit (1-1) is a constituent unit having a structure represented by the following general formula (I-a) or (I-b), or a structure of an acid addition salt thereof:

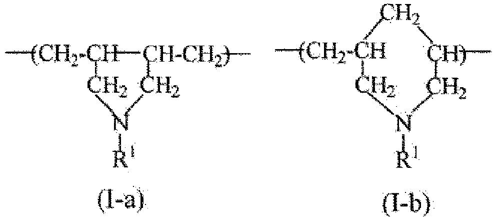

In the formula, R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms. R¹ represents preferably a hydrogen atom, a methyl group, an ethyl group, or a benzyl group, and particularly preferably a hydrogen atom, or a methyl group.

The constituent unit (1-1) may have a structure that is an inorganic acid salt, an organic acid salt, or the like of the structure represented by the above structural formula (1-a) or (1-b), that is, a structure of an acid addition salt thereof.

The specific amphoteric copolymer has a constituent unit (1-1), and from the viewpoint of production costs, and the like, a diallylamine monomer having an addition salt is preferably used in producing the specific amphoteric copolymer. Since a process of removing the addition salt such as HCl from the polymer is complicated and causes an increase in cost, it is a preferable embodiment also from the viewpoint of costs and the like to use an addition salt-type constituent unit (1-1) with which the specific amphoteric copolymer can be produced without such a process.

From the viewpoint of the ease of availability, the controllability of reaction, and the like, the inorganic acid salt or organic acid salt in the constituent unit (1-1) of this embodiment is preferably a hydrochloride, a carboxylate, a sulfonate, or an alkyl sulfate, and particularly preferably a hydrochloride.

### Constituent unit (1-4)

The constituent unit (1-4) is a constituent unit having a structure represented by the following general formula (I-f), or a structure of an acid addition salt thereof:

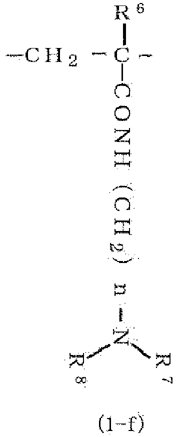

In the formula, R⁶ represents a hydrogen atom or a methyl group, R⁷ and R⁸ each independently represent an alkyl group having 1 to 4 carbon atoms, and n is an integer of 2 to 4.

R⁶ represents preferably a methyl group, n represents preferably 2 to 3, and R⁷ and R⁸ are each preferably a methyl group.

There is no particular limitation on the type of an addition salt in a case where the constituent unit (1-4) is an acid addition salt having a structure represented by general formula (I-f), but from the viewpoint of the ease of availability, controllability of reaction, and the like, for example, a hydrochloride, a sulfate, a phosphate, a nitrate, a sulfite, a phosphite, a nitrite, a hydrobromide, an acetate, an amide sulfate, a methanesulfonate, a trifluoroacetate, a p-toluenesulfonate, or the like can be used.

Among them, a hydrochloride, a sulfate, a phosphate, and an amide sulfate are preferable, and a hydrochloride, a sulfate, a phosphate and an amide sulfate which have a structure derived from monoallylamine, are particularly preferable.

The proportion of a cationic constituent unit (1) to the whole constituent units of the specific amphoteric copolymer is usually 10 to 70 mol%, preferably 15 to 60 mol%, and particularly preferably 20 to 55 mol%. In a case where two or more types of cationic constituent units (1) are contained, the proportion of the unit is defined based on the total amount of the two or more types of cationic constituent units (1).

### Anionic constituent unit (2)

The anionic constituent unit (2) constituting the specific amphoteric copolymer is at least one type of anionic constituent unit selected from the group consisting of constituent units (2-1) and (2-4).

### Constituent unit (2-1)

A constituent unit (2-1) is a constituent unit having a structure represented by the following general formula (II-a):

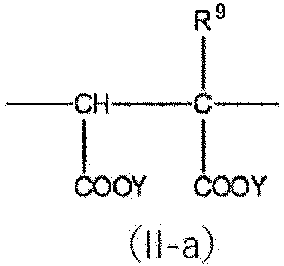

In the formula, R⁹ represents a hydrogen or a methyl group, Y represents a hydrogen, Na, K, NH₄, 1/2Ca, 1/2Mg, 1/2Fe, 1/3Al, or 1/3Fe independently for each carboxy group to be bonded.

R⁹ represents preferably a hydrogen, and Y represents preferably a hydrogen or Na. The constituent unit (2-1) is particularly preferably derived from maleic acid.

### Constituent unit (2-4)

A constituent unit (2-4) is a constituent unit having a structure represented by the following general formula (II-d):

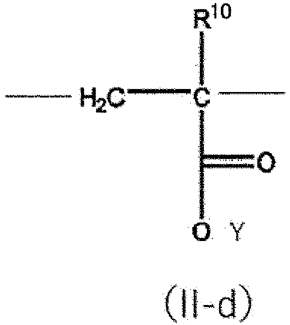

In the formula, R¹⁰ is a hydrogen or a methyl group, and Y represents a hydrogen, Na, K, NH₄, 1/2Ca, 1/2Mg, 1/2Fe, 1/3Al, or 1/3Fe independently for each carboxy group to be bonded.

R¹⁰ represents preferably a hydrogen, and Y represents preferably a hydrogen or Na.

The constituent unit (2-4) is preferably derived from (meth)acrylic acid, and particularly preferably derived from acrylic acid.

The specific amphoteric copolymer comprises a cationic constituent unit (1), as described above, and an anionic constituent unit (2), as described above, and thus possesses cationicity and anionicity.

The specific amphoteric copolymer, which comprises a cationic constituent unit (1) having a specific structure, as described above, and an anionic constituent unit (2) having a specific structure, as described above, significantly increases a difference in the melting temperature of a double-stranded nucleic acid between the presence and absence of double-stranded nucleic acid mismatch. Although not adhering to a theory, the mechanism for bringing about the remarkable effect may be due to as follows: If the cationic density of a cationic constituent unit(s) is too high, the bond between nucleic acids becomes too strong, which makes it difficult to measure a double-stranded nucleic acid melting temperature. On the other hand, if the cationic density of a cationic constituent unit(s) is too low, the rise in the melting temperature of a double-stranded nucleic acid becomes small and the detection sensitivity of mismatch decreases. It is thus inferred that the positive charge of a cationic constituent unit (1) having a specific structure, as described above, and the negative charge of an anionic constituent unit (2) having a specific structure, as described above, moderately interfere with each other, and the bond between nucleic acids is moderately strengthened.

As described above, since the mismatching in a double-stranded nucleic acid or the mutation with respect to a standard nucleic acid in a sample nucleic acid is more reliably detectable, in the specific amphoteric copolymer, the cationic constituent unit (1) is selected from the group consisting of constituent units (1-1) and (1-4), and the anionic constituent unit (2) is selected from the group consisting of constituent units (2-1) and (2-4).

The proportion of the total of the cationic constituent unit (1) and the anionic constituent unit (2) to the whole constituent units of a specific amphoteric copolymer is not particularly limited, but is usually 25 mol% or more, preferably 30 to 90 mol%, more preferably 35 to 75 mol%, and particularly preferably 40 to 60 mol%.

The proportion of the cationic constituent unit (1) and the anionic constituent unit (2) is also not particularly limited, but is usually 0.1 : 1 to 2 : 1, preferably 0.3 : 1 to 0.8 : 1, more preferably 0.4 : 1 to 1.2 : 1, and particularly preferably 0.5 : 1 to 1 : 1 in terms of molar ratio (cationic constituent unit (1) : anionic constituent unit (2)).

In a case where the structures of the cationic constituent unit (1) and the anionic constituent unit (2) are known, the molar ratio of the cationic constituent unit (1) and anionic constituent unit (2) in the specific amphoteric copolymer can be specified by reprecipitating the specific amphoteric copolymer with an organic solvent such as isopropyl alcohol, or acetone, and by analyzing the reprecipitation in an appropriate mode according to the structure of the constituent unit, using Perkin Elmer 2400II CHNS/O fully automatic elemental analyzer or a device with a performance equivalent thereto. In addition, the measurement can be performed by using helium gas as a carrier gas, weighing a solid sample into a tin capsule, dropping the sample into a combustion tube to burn the sample at a combustion temperature of 1800°C or more in pure oxygen gas, detecting each component to be measured by a frontal chromatography system with a separation column and a thermal conductivity detector, and quantifying the content of each element using a calibration factor. In this regard, in a case where the structures of the cationic constituent unit (1) and anionic constituent unit (2) in the specific amphoteric copolymer are unknown, the structures of the respective constituent units are specified by a known method using 1H-NMR or 13C-NMR before the measurement by the elemental analyzer. Further, the calculation can be performed with the amount of each monomer supplied in the production (copolymerization) of the specific amphoteric copolymer, and the amount of each monomer remaining without being incorporated into the specific amphoteric copolymer. In this regard, since the proportion (molar ratio) of the constituent unit derived from each monomer in the specific amphoteric copolymer is almost the same as the charge composition (molar ratio) of each constituent unit, the mixing ratio of a monomer may be treated as the proportion (molar ratio) of a constituent unit for convenience in the present specification.

### Other constituent units

The specific amphoteric copolymer may comprise other constituent units in addition to a cationic constituent unit (1) and an anionic constituent unit (2), described above.

Examples of the other constituent units include a nonionic constituent unit (3), and a cationic or anionic constituent unit that does not correspond to any of the cationic constituent units (1) and the anionic constituent unit (2).

The specific amphoteric copolymer preferably comprises a nonionic constituent unit (3). A nonionic constituent unit (3) can be positioned between a cationic constituent unit (1) and an anionic constituent unit (2) to function as a spacer between the cationic constituent unit (1) and the anionic constituent unit (2) and control the interference between the positive and negative charges of each constituent unit, which allows a mismatch in a double-stranded nucleic acid or a mutation in a sample nucleic acid with respect to a standard nucleic acid to be more reliably detectable. Further, a nonionic constituent unit is preferably positioned between a cationic constituent unit (1) and an anionic constituent unit (2). In particular, the specific amphoteric copolymer particularly preferably comprises a nonionic constituent unit (3) in a case where a cationic constituent unit has a structure represented by general formula (I-f) or a structure of an acid addition salt thereof. In that case, a nonionic constituent unit particularly effectively functions as a spacer.

### Nonionic constituent unit (3)

A nonionic constituent unit (3) in the present embodiment may be a constituent unit derived from a nonionic monomer that can be copolymerized with a cationic constituent unit (1) and an anionic constituent unit (2), and is not otherwise particularly limited, but as the nonionic constituent unit (3), a constituent unit derived from a methacrylic acid ester-based monomer, an acrylic ester-based monomer, a methacrylamide-based monomer, an acrylamide-based monomer, sulfur dioxide, or the like can be preferably used. More specific examples of the nonionic constituent unit (3) include constituent units derived from methyl methacrylate, ethyl methacrylate, butyl methacrylate, methyl acrylate, ethyl acrylate, butyl acrylate, methacrylamide, N-methyl methacrylamide, dimethylmethacrylamide, N-(3-dimethylaminopropyl)methacrylamide, acrylamide, dimethylacrylamide, hydroxyethyl acrylamide, dimethylaminopropyl acrylamide, a dimethylaminopropyl acrylamide methyl chloride quaternary salt, acryloylmorpholine, isopropyl acrylamide, 4-t-butylcyclohexyl acrylate, and sulfur dioxide. It is particularly preferable to use a constituent unit derived from acrylamide.

The nonionic constituent unit (3) can usually be introduced into a polymer by using a nonionic monomer as the monomer.

There is no particular limitation on the content of the nonionic constituent unit (3) in a case where the specific amphoteric copolymer has the nonionic constituent unit (3), and the suitable amount varies depending on the type of the nonionic constituent unit (3), but the molar ratio with the anionic constituent unit (2), that is, nonionic constituent unit (3)/anionic constituent unit (2) = preferably 0.1/1 to 1/1, more preferably 0.2/1 to 0.8/1, furthermore preferably, 0.3/1 to 0.7/1, and particularly preferably 0.4/1 to 0.6/1.

In a case where the nonionic constituent unit (3) is derived from a methacrylic acid ester-based monomer, an acrylic ester-based monomer, a methacrylamide-based monomer, or an acrylamide-based monomer, the above molar ratio is preferably 0.1/1 to 1/1, more preferably 0.2/1 to 0.8/1, furthermore preferably, 0.3/1 to 0.7/1, and particularly preferably 0.4/1 to 0.6/1.

In a case where the nonionic constituent unit (3) is derived from sulfur dioxide, the above molar ratio is preferably 0.1/1 to 1/1, and particularly preferably 0.2/1 to 1/1.

### Production method of specific amphoteric copolymer

There is no particular limitation on the production method of a specific amphoteric copolymer, described above, and the specific amphoteric copolymer can be produced by a method conventionally known in the technical field to which the invention belongs, and for example, the specific amphoteric copolymer can be produced by copolymerizing a cationic monomer having a structure corresponding to the cationic constituent unit (1), an anionic monomer having a structure corresponding to the anionic constituent unit (2), and if desired, a monomer such as a nonionic monomer having a structure corresponding to the nonionic constituent unit (3).

A solvent in a case of copolymerizing a cationic monomer having a structure corresponding to the cationic constituent unit (1), an anionic monomer having a structure corresponding to the anionic constituent unit (2), and the like is not particularly limited, and may be a water-based solvent, or an organic solvent such as alcohol, ether, sulfoxide, or amide, but is preferably a water-based solvent.

A concentration of the monomer in the case of copolymerizing a cationic monomer having a structure corresponding to the cationic constituent unit (1), an anionic monomer having a structure corresponding to the anionic constituent unit (2), and the like varies depending on the type of the monomer and the type of the solvent used for the copolymerization, but is usually 10 to 75% by mass in a case of a water-based solvent. This copolymerization reaction is usually a radical polymerization reaction, and is performed in the presence of a radical polymerization catalyst. The type of the radical polymerization catalyst is not particularly limited, and preferable examples of the radical polymerization catalyst include a peroxide such as t-butyl hydroperoxide, a persulfate such as ammonium persulfate, sodium persulfate, or potassium persulfate, and a water-soluble azo compound such as an azobis-based compound, or a diazo-based compound.

The amount of the radical polymerization catalyst to be added is commonly 0.1 to 20 mol%, and preferably 1.0 to 10 mol% based on the whole monomers. The polymerization temperature is commonly 0 to 100°C and preferably 5 to 80°C, and the polymerization time is commonly 1 to 150 hours and preferably 5 to 100 hours. As the polymerization atmosphere, even in the air, there is no major problem in polymerization, and the polymerization can be performed in an atmosphere of an inert gas such as nitrogen.

### 2. Method for detecting nucleic acid mismatching between sample nucleic acid and probe nucleic acid

Another embodiment of the present invention relates to a method for detecting nucleic acid mismatching between a sample nucleic acid and a probe nucleic acid, comprising the steps of:
(1) measuring melting temperatures of a double-stranded nucleic acid between the probe nucleic acid and a nucleic acid having a base sequence complementary to the probe nucleic acid with and without adding an agent for raising a melting temperature of a double-stranded nucleic acid, and determining a rise value between the melting temperatures of the probe nucleic acid and the nucleic acid having a base sequence complementary to the probe nucleic acid due to the presence of the amphoteric copolymer (standard ΔTm);
(2) measuring melting temperatures of a double-stranded nucleic acid between the sample nucleic acid and the probe nucleic acid with and without adding an agent for raising a melting temperature of a double-stranded nucleic acid, and determining a rise value between the melting temperatures of the double-stranded nucleic acid of the sample nucleic acid and the probe nucleic acid due to the presence of the specific amphoteric copolymer (sample ΔTm); and
(3) determining the presence or absence of nucleic acid mismatching based on a value (ΔΔTm) obtained by subtracting the sample ΔTm from the standard ΔTm,
   wherein the agent for raising a melting temperature of a double-stranded nucleic acid comprises:
   an amphoteric copolymer, wherein the amphoteric copolymer comprises:
   at least one kind of cationic constituent unit (1) selected from the group consisting of
   a constituent unit (1-1) having a structure represented by general formula (I-a) or general formula (I-b), or a structure of an acid addition salt thereof: 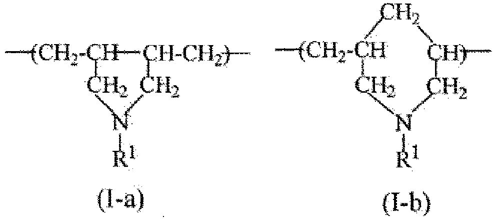
   wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
   a constituent unit (1-4) having a structure represented by general formula (1-f), or a structure of an acid addition salt thereof: 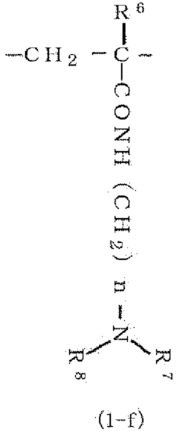
   wherein R⁶ represents a hydrogen atom or a methyl group, R⁷ and R⁸ each independently represent an alkyl group having 1 to 4 carbon atoms, and n is an integer of 2 to 4; and
   at least one kind of anionic constituent unit (2) selected from the group consisting of
   a constituent unit (2-1) having a structure represented by general formula (II-a): 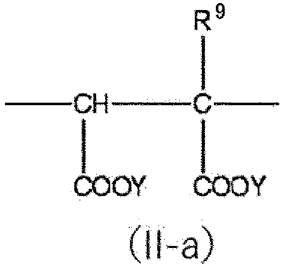
   wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
   a constituent unit (2-4) having a structure represented by general formula (II-d): 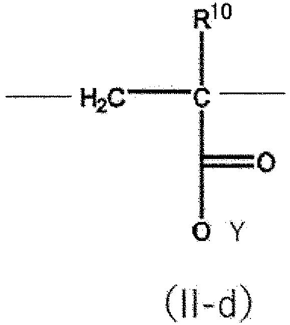
   wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

In step (1), the rise value (standard ΔTm) between the melting temperatures is determined by measuring a melting temperature (Tm1) of a double-stranded nucleic acid of the probe nucleic acid and a nucleic acid having a base sequence complementary to the probe nucleic acid in the presence of the specific amphoteric copolymer, and a melting temperature (Tm2) of a double-stranded nucleic acid of the probe nucleic acid and a nucleic acid having a base sequence complementary to the probe nucleic acid in the absence of the specific amphoteric copolymer, respectively, and by calculating Tm1 - Tm2.

Similarly, in step (2), the rise value (sample ΔTm) between the melting temperatures is determined by measuring a melting temperature (Tm1) of a double-stranded nucleic acid of the sample nucleic acid and the probe nucleic acid in the presence of the specific amphoteric copolymer, and a melting temperature (Tm2) of a double-stranded nucleic acid of the sample nucleic acid and the probe nucleic acid in the absence of the specific amphoteric copolymer, and by calculating Tm1 - Tm2.

In the specification of the present application, the term "nucleic acid" includes both DNA and RNA. The "nucleic acid" also includes an artificial nucleic acid. The sample nucleic acid and the nucleic acid having a sequence complementary to the probe nucleic acid may include any nucleic acid such as genomic DNA, cDNA, genomic RNA, mRNA, or rRNA. Further, the probe nucleic acid and the nucleic acid having a sequence complementary to the probe nucleic acid may be a natural nucleic acid or an artificial nucleic acid. The artificial nucleic acid is DNA or RNA in part of which an artificial nucleotide is contained, and examples of the artificial nucleotide include BNA (bridged nucleic acid), LNA (locked nucleic acid), PNA (peptide nucleic acid), a peptide nucleic acid (PHONA) having a phosphate group, and a nucleic acid in at least part of which a morpholino nucleic acid is contained.

The polymerization degree of the sample nucleic acid and the probe nucleic acid is not particularly limited, and is, from the point of detection sensitivity, usually 4 to 100 mers, preferably 5 to 50 mers, more preferably 6 to 30 mers, furthermore preferably 7 to 20 mers, and particularly preferably 8 to 15 mers.

The GC content of the sample nucleic acid and the probe nucleic acid is not particularly limited, and is, from the point of detection sensitivity, usually 30 to 80%, and preferably 50 to 75%.

The melting temperature of a double-stranded nucleic acid is usually measured by using a nucleic acid aqueous solution, and the solvent to be used may be a nuclease-free aqueous medium and is typically nuclease-free water. Further, the nucleic acid may be dissolved in a buffer solution, and as the buffer solution, for example, a buffer solution for nucleic acids used in a nucleic acid analysis reagent can be used. Further, the nucleic acid aqueous solution may contain a surfactant within the range of not affecting the rise in melting temperature (Tm) due to the specific amphoteric copolymer, and as the surfactant, a surfactant used in a nucleic acid analysis reagent can be mentioned.

In the measurement of the melting temperature (Tm) of a double-stranded nucleic acid, the specific amphoteric copolymer can be contained in a solution usually at a concentration of 0.03 to 3.0% by mass, and preferably at a concentration of 0.2 to 2.0% by mass.

In one embodiment of the present disclosure, in addition to the specific amphoteric copolymer, a known agent for raising a melting temperature (Tm) such as MgCl₂ may be contained in the reaction mixture. However, in a case of containing MgCl₂, the concentration is preferably 5 mM or less. Further, a salt such as NaCl may be contained within the range of not affecting the rise in melting temperature (Tm). In this case, the salt is preferably 300 mM or less.

In the measurement of the melting temperature (Tm) of a double-stranded nucleic acid, the sample nucleic acid and the probe nucleic acid each can be measured usually at a concentration of 10 to 1000 µM, and preferably at a concentration of 30 to 300 µM.

The solution pH can be set to 3.0 to 11.0, and preferably 5.0 to 9.0.

The temperature schedule in the measurement of a melting temperature (Tm) of a double-stranded nucleic acid varies depending on the method for detecting denaturation of the double-stranded nucleic acid, but basically, the temperature is allowed to gradually increase from the temperature at which the double strand is completely formed so that the denaturation of the double-stranded nucleic acid is caused.

Examples of the method for detecting the denaturation of a double-stranded nucleic acid include: a method of utilizing the fact that the absorbance of a nucleic acid in a UV range (for example, 260 nm) changes with the denaturation of the double-stranded nucleic acid; a method for detecting the degree of formation of the double-stranded nucleic acid by using a probe nucleic acid labeled with a fluorescent dye, enzyme, a light emitting dye, or the like, or a probe nucleic acid and a nucleic acid having a sequence complementary to the probe nucleic acid (for example, FRET method); and a method of utilizing the decrease in fluorescence intensity with the denaturation of the double-stranded nucleic acid by incorporating a fluorescent dye or the like between nucleic acids at the time of formation of the double-stranded nucleic acid (for example, intercalator method).

From the point that there is no need to prepare a probe nucleic acid labeled with a fluorescent dye or the like and real-time analysis is possible with a simple device, a method of utilizing the decrease in fluorescence intensity with the denaturation of the double-stranded nucleic acid by incorporating a fluorescent dye or the like between nucleic acids at the time of formation of the double-stranded nucleic acid (for example, intercalator method) is preferable. In addition, when referring to a specific numerical value of Tm in the specification of the present application, unless otherwise particularly specified by another method, the specific numerical value means a numerical value measured by the method described in Example 1, and specific numerical values of standard ΔTm, sample ΔTm, and ΔΔTm mean the values determined by calculation from the Tm thus obtained.

The detection sensitivity of nucleic acid mismatching between a sample nucleic acid and a probe nucleic acid may vary depending on conditions such as the length of the nucleic acid, the GC content of the nucleic acid, and the salt concentration, but in the detection method of the present invention, if the polymerization degree of the nucleic acid is 20 or less, even if there is mismatching of one base, the standard ΔTm is usually higher than the sample ΔTm by 5.0°C or more. Therefore, in a case where the standard ΔTm is higher than the sample ΔTm by 5.0°C or more, it is preferable to determine that double-stranded nucleic acid mismatching is present, and from the viewpoint of reducing false positives, preferably, in a case where the standard ΔTm is higher than the sample ΔTm by 7.5°C or more, it is more preferable to determine that nucleic acid mismatching is present, and in a case where the standard ΔTm is higher than the sample ΔTm by 10.0°C or more, it is particularly preferable to determine that nucleic acid mismatching is present.

### 3. Method for detecting mutation with respect to standard nucleic acid in sample nucleic acid

As one embodiment of the method for detecting nucleic acid mismatching, the presence or absence of mutation in a sample nucleic acid with respect to a standard nucleic acid (for example, wild-type genomic nucleic acid) can be detected. Specifically, by using an antisense strand of a wild-type genomic nucleic acid as the probe nucleic acid in the above detection method and a sense strand of the wild-type genomic nucleic acid as the nucleic acid having a base sequence complementary to the probe nucleic acid, and by detecting nucleic acid mismatching between the probe nucleic acid, that is, the antisense strand of the wild-type genomic nucleic acid, and the sample nucleic acid in the same way as above, the mutation with respect to the wild-type genomic nucleic acid in the sample nucleic acid can be detected. In other words, another embodiment of the present invention provides a method for detecting mutation with respect to a standard nucleic acid in a sample nucleic acid, comprising the steps of:
(1) measuring melting temperatures of a double-stranded nucleic acid between the standard nucleic acid and a nucleic acid having a base sequence complementary to the standard nucleic acid with and without adding an agent for raising a melting temperature of a double-stranded nucleic acid, and determining a rise value (standard ΔTm) between the melting temperatures of the double-stranded nucleic acid between the standard nucleic acid and the nucleic acid having the base sequence complementary to the standard nucleic acid due to the presence of the amphoteric copolymer;
(2) measuring melting temperatures of a double-stranded nucleic acid of the sample nucleic acid and the nucleic acid having a base sequence complementary to the standard nucleic acid with and without adding an agent for raising a melting temperature of a double-stranded nucleic acid, and determining a rise value (sample ΔTm) between the melting temperatures of the double-stranded nucleic acid between the sample nucleic acid and the nucleic acid having the base sequence complementary to the standard nucleic acid due to the presence of the amphoteric copolymer; and
(3) determining the presence or absence of mutation based on a value (ΔΔTm) obtained by subtracting the sample ΔTm from the standard ΔTm,
   wherein the agent for raising a melting temperature of a double-stranded nucleic acid comprises:
   an amphoteric copolymer, wherein the amphoteric copolymer comprises:
   at least one kind of cationic constituent unit (1) selected from the group consisting of
   a constituent unit (1-1) having a structure represented by general formula (I-a) or general formula (I-b), or a structure of an acid addition salt thereof: 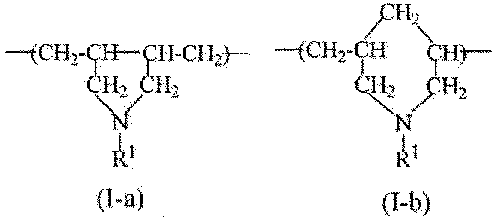
   wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
   a constituent unit (1-4) having a structure represented by general formula (1-f), or a structure of an acid addition salt thereof: 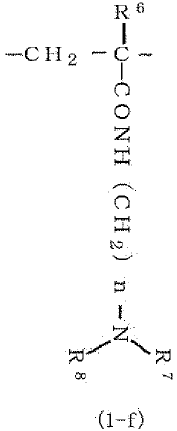
   wherein R⁶ represents a hydrogen atom or a methyl group, R⁷ and R⁸ each independently represent an alkyl group having 1 to 4 carbon atoms, and n is an integer of 2 to 4; and
   at least one kind of anionic constituent unit (2) selected from the group consisting of
   a constituent unit (2-1) having a structure represented by general formula (II-a): 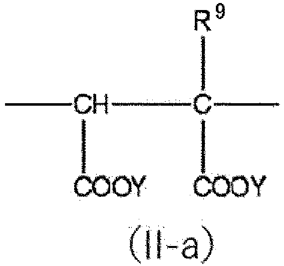
   wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
   a constituent unit (2-4) having a structure represented by general formula (II-d): 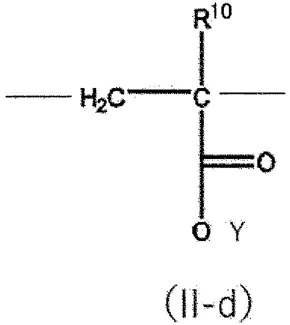
   wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

### 4. Kit for detecting nucleic acid mismatching and kit for detecting mutation

Another embodiment of the present disclosure provides kits for carrying out the above-described method for detecting nucleic acid mismatching between a sample nucleic acid and a probe nucleic acid and method for detecting mutation with respect to a standard nucleic acid in a sample nucleic acid. Specifically, one embodiment provides
a kit for detecting nucleic acid mismatching between a sample nucleic acid and a probe nucleic acid, comprising:
the probe nucleic acid;
a nucleic acid having a base sequence complementary to the probe nucleic acid; and
an agent for raising a melting temperature of a double-stranded nucleic acid,
wherein the agent for raising a melting temperature of a double-stranded nucleic acid comprises:
   an amphoteric copolymer, wherein the amphoteric copolymer comprises:
   at least one kind of cationic constituent unit (1) selected from the group consisting of
   a constituent unit (1-1) having a structure represented by general formula (I-a) or general formula (I-b), or a structure of an acid addition salt thereof: 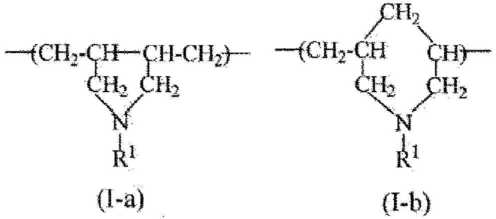
   wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
   a constituent unit (1-4) having a structure represented by general formula (1-f), or a structure of an acid addition salt thereof: 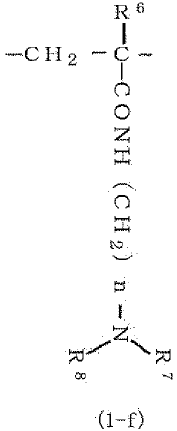
   wherein R⁶ represents a hydrogen atom or a methyl group, R⁷ and R⁸ each independently represent an alkyl group having 1 to 4 carbon atoms, and n is an integer of 2 to 4; and
   at least one kind of anionic constituent unit (2) selected from the group consisting of
   a constituent unit (2-1) having a structure represented by general formula (II-a): 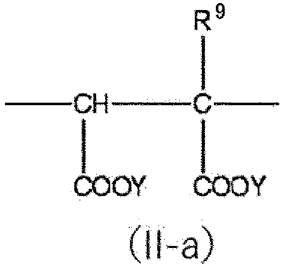
   wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
   a constituent unit (2-4) having a structure represented by general formula (II-d): 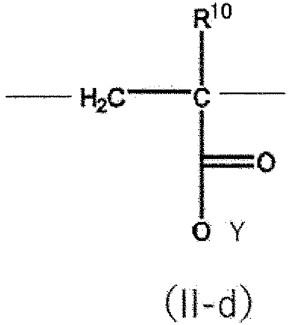
   wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

Further, another embodiment provides
a kit for detecting mutation between a sample nucleic acid and a standard nucleic acid, comprising:
the standard nucleic acid;
a nucleic acid having a base sequence complementary to the standard nucleic acid; and
an agent for raising a melting temperature of a double-stranded nucleic acid,
wherein the agent for raising a melting temperature of a double-stranded nucleic acid comprises:
   an amphoteric copolymer, wherein the amphoteric copolymer comprises:
   at least one kind of cationic constituent unit (1) selected from the group consisting of
   a constituent unit (1-1) having a structure represented by general formula (I-a) or general formula (I-b), or a structure of an acid addition salt thereof: 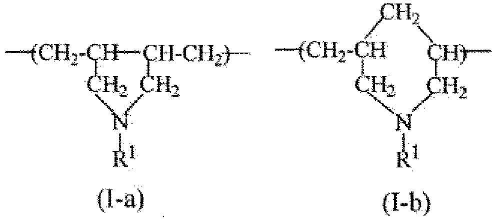
   wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
   a constituent unit (1-4) having a structure represented by general formula (1-f), or a structure of an acid addition salt thereof: 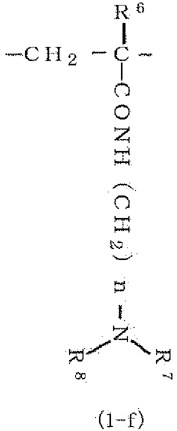
   wherein R⁶ represents a hydrogen atom or a methyl group, R⁷ and R⁸ each independently represent an alkyl group having 1 to 4 carbon atoms, and n is an integer of 2 to 4; and
   at least one kind of anionic constituent unit (2) selected from the group consisting of
   a constituent unit (2-1) having a structure represented by general formula (II-a): 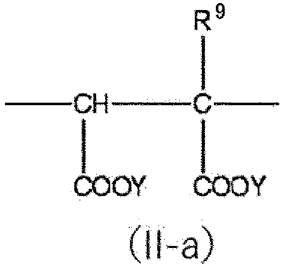
   wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
   a constituent unit (2-4) having a structure represented by general formula (II-d): 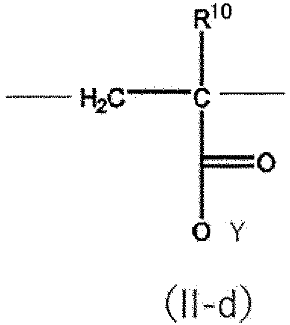
   wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

In these kits, details and preferred forms of the "probe nucleic acid", "nucleic acid having a base sequence complementary to the probe nucleic acid", "standard nucleic acid", "nucleic acid having a base sequence complementary to the standard nucleic acid", and "specific amphoteric copolymer" are as described above.

Further, it is also as described above that in addition to them, other components that can be used to measure a melting temperature (Tm) of a double-stranded nucleic acid, such as an aqueous medium, a buffer solution, a surfactant, a salt, other agents for raising a melting temperature (Tm), and a reagent for detecting a double-stranded nucleic acid may also be contained. In particular, from the point of facilitating the detection of nucleic acid mismatching and the detection of mutation, as the reagent for detecting a double-stranded nucleic acid, it is preferable to contain an intercalating fluorescent dye.

### EXAMPLES

Hereinafter, the present invention is further described by Examples, however, the present invention is not limited to the Examples.

### [Example 1]

A dimethylamino propyl methacrylamide hydrochloride/acrylamide/acrylic acid copolymer was evaluated for detection of mismatching by comparing a Tm rising range by a mismatch double strand with a Tm rising range by a full-match double strand with a polymer aqueous solution containing the copolymer as an agent for raising a Tm of a double-stranded nucleic acid.

### 1. Polymer aqueous solution (agent for raising Tm of double-stranded nucleic acid)

As an agent for raising a Tm of a double-stranded nucleic acid, a polymer aqueous solution having a pH of 7.0 and containing 10.0 % by mass of a dimethylamino propyl methacrylamide hydrochloride/acrylamide/acrylic acid copolymer was prepared. The copolymer was obtained by copolymerizing a dimethylamino propyl methacrylamide hydrochloride (monomer 1), acrylamide (monomer 2), and acrylic acid (monomer 3) under the following polymerization condition 1 and has a molar ratio of a constituent unit 1 derived from monomer 1, a constituent unit 2 derived from monomer 2, and a constituent unit 3 derived from monomer 3 of 1 : 1 : 2.

Polymerization condition 1: Into a 300-mL four-neck flask equipped with a thermometer, a stirrer, and a cooling pipe, 16.15 g (equivalent to 0.04 mol) of a dimethylamino propyl methacrylamide hydrochloride (solid content concentration:51.2 % by mass), 2.93 g (equivalent to 0.04 mol) of acrylamide (solid content concentration: 97 % by mass), 5.82 g (equivalent to 0.08 mol) of acrylic acid (solid content concentration: 99% by mass), and 143.86 g of distilled water were charged, and heated to 60°C. Every 2 hours, in the aqueous solution, 28.5% by mass of ammonium persulfate aqueous solution was added at such an amount that the amount of ammonium persulfate was 0.25, 0.5, 0.5, and 0.75 mol%, respectively to the whole amount of monomers, and the reaction was continued overnight.

### 2. Standard nucleic acid, nucleic acid consisting of complementary sequence, and nucleic acid having mutation

In order to evaluate whether or not mutation at one base with respect to the nucleotide sequence of a standard nucleic acid is detectable, the following oligo DNAs were used.

**[Table 1]**

| | | |
|---|---|---|
| Reference nucleic acid | 11-mer oligo DNA derived from part of Exon 2 of wild-type KRAS gene sense strand | GGTG**G**CGTAGG (SEQ ID NO: 1) |
| Nucleic acid consisting of sequence complementary to reference nucleic acid (also referred to as complementary strand) | 11-mer oligo DNA of antisense strand consisting of sequence complementary to the sense strand oligo DNA | CCTACG**C**CACC (SEQ ID NO: 2) |
| Nucleic acid having mutation with respect to reference nucleic acid (also referred to as mutation strand) | 11-mer oligo DNA having mutation at one base with respect to the sense strand DNA oligomer | GGTG**A**CGTAGG (SEQ ID NO: 3) |

| | | |
|---|---|---|
| In a sequence of each oligo DNA in the table, a mismatch site is indicated with bold letter. | | |

### 3. Preparation of reaction mixture

The following reaction mixtures 1 to 4 were prepared. A fluorescent dye in a fluorescent dye solution corresponds to an intercalating fluorescent dye.

**[Table 2]**

| Reaction mixture 1 | |
|---|---|
| Components | Content |
| 500 µM of reference nucleic acid aqueous solution | 4.0 *µ*L |
| 500 µM of complementary strand aqueous solution | 4.0 *µ*L |
| 25 µM of fluorescent dye solution (manufactured by Biotium, trade name: EvaGreen) | 1.0 *µ*L |
| Nuclease-free water | 11.0 *µ*L |

### Reaction mixture 2

| Components | Content |
|---|---|
| 500 µM of reference nucleic acid aqueous solution | 4.0*µ*L |
| 500 µM of complementary strand aqueous solution | 4.0 *µ*L |
| 25 µM of fluorescent dye solution (manufactured by Biotium, trade name: EvaGreen) | 1.0 *µ*L |
| 10.0 % by mass of polymer aqueous solution | 2.0*µ*L |
| Nuclease-free water | 9.0 *µ*L |

### Reaction mixture 3

| Components | Content |
|---|---|
| 500 µM of mutation strand aqueous solution | 4.0*µ*L |
| 500 µM of complementary strand aqueous solution | 4.0 *µ*L |
| 25 µM of fluorescent dye solution (manufactured by Biotium, trade name: EvaGreen) | 1.0 *µ*L |
| Nuclease-free water | 11.0*µ*L |

### Reaction mixture 4

| Components | Content |
|---|---|
| 500 µM of mutation strand aqueous solution | 4.0*µ*L |
| 500 µM of complementary strand aqueous solution | 4.0*µ*L |
| 25 µM of fluorescent dye solution (manufactured by Biotium, trade name: EvaGreen) | 1.0 *µ*L |
| 10.0 % by mass of polymer aqueous solution | 2.0*µ*L |
| Nuclease-free water | 9.0 *µ*L |

Concentration of the polymer (agent for raising Tm of a double-stranded nucleic acid) in reaction mixtures 2 and 4 was 1.0 % by mass.

### 4. Creation of double-stranded melting curve

Each of the prepared reaction mixtures was set in StepOnePlus Real-Time PCR System (manufactured by Thermo Fisher Scientific), heated at 95°C for 15 seconds, and then kept at 10°C for 1 minute. Next, while performing fluorescence measurement every 0.3 seconds, the temperature was raised to 95°C to obtain a double-stranded melting curve, and then held at 95°C for 15 seconds.

### [Standard Example]

As a standard, a double-stranded melting curve was obtained with a magnesium chloride solution that is conventionally known as an agent for raising a Tm of a double-stranded nucleic acid, in place of the polymer aqueous solution.

Specifically, the following reaction mixtures 5 and 6 were prepared in place of the reaction mixtures 2 and 4, and a double-stranded melting curve was obtained from each reaction mixture in a similar manner to Example 1.

**[Table 3]**

| Reaction mixture 5 | |
|---|---|
| Components | Content |
| 500 µM of reference nucleic acid aqueous solution | 4.0 *µ*L |
| 500 µM of complementary strand aqueous solution | 4.0 *µ*L |
| 25 µM of fluorescent dye solution (manufactured by Biotium, trade name: EvaGreen) | 1.0 *µ*L |
| 15 mM MgCl₂ aqueous solution | 2.0*µ*L |
| Nuclease-free water | 9.0 *µ*L |

### Reaction mixture 6

| 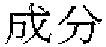 | Content |
|---|---|
| 500 µM of mutation strand aqueous solution | 4.0 *µ*L |
| 500 µM of complementary strand aqueous solution | 4 .0 *µ*L |
| 25 µM of fluorescent dye solution (manufactured by Biotium, trade name: EvaGreen) | 1.0*µ*L |
| 15 mM MgCl₂ aqueous solution | 2. 0*µ*L |
| Nuclease-free water | 9. 0*µ*L |

### [Test Results]

Double-stranded melting curves obtained from the reaction mixtures 1, 2 and 5 are shown in FIG. 1, and double-stranded melting curves obtained from the reaction mixtures 3, 4 and 6 are shown in FIG. 2. As shown in FIG. 1, a Tm value of a double-strand between the standard nucleic acid and the complementary strand was 37.0°C in the absence of the polymer (agent for raising Tm of a double-stranded nucleic acid). On the other hand, a Tm value of a double-strand between the standard nucleic acid and the complementary strand was 67.30°C in the presence of the polymer (agent for raising Tm of a double-stranded nucleic acid), and a 30.30°C-rise in the Tm value was observed.

Further, as shown in FIG. 2, a Tm value of a double-strand between the mutation strand and the complementary strand was 34.25°C in the absence of the polymer (agent for raising Tm of a double-stranded nucleic acid). On the other hand, a Tm value of a double-strand between the mutation strand and the complementary strand was 53.35°C in the presence of the polymer (agent for raising Tm of a double-stranded nucleic acid), and a 19.10°C-rise in the Tm value was observed.

As a result, a difference of 11.20°C was observed between the rise in the Tm value of a double-strand between the standard nucleic acid and the complementary strand and the rise in the Tm value of a double-strand between the mutation strand and the complementary strand in the presence of the polymer (agent for raising Tm of a double-stranded nucleic acid).

### [Example 2]

A melting curve of a double strand was obtained from each reaction mixture in a similar manner to Example 1 except that a diallylamine hydrochloride/maleic acid copolymer obtained by copolymerizing a diallylamine hydrochloride (monomer 1) and maleic acid (monomer 2) under the following polymerization condition 2 and having a molar ratio of a constituent unit 1 derived from the monomer 1 and a constituent unit 2 derived from the monomer 2 of 1 : 1 was used as the agent for raising a Tm of a double-stranded nucleic acid, and then the Tm rising range (standard ΔTm) between a standard nucleic acid and a complementary strand and Tm rising range (sample ΔTm) between a mutation strand and a complementary strand due to the presence of a polymer (agent for raising a Tm of a double-stranded nucleic acid) were determined, and the difference (ΔΔTm) between them was calculated.

Polymerization condition 2: Into a 500-mL four-neck flask equipped with a thermometer, a stirrer, and a cooling pipe, 160.07 g (0.80 mol) of 66.78% diallylamine hydrochloride, 78.45 g (0.80 mol) of maleic anhydride, and 91.86 g of distilled water were charged, and the internal temperature was raised to 50°C. 28.5% by mass of ammonium persulfate aqueous solution was added at such an amount that the amount of ammonium persulfate in the aqueous solution was 0.5% by mass to the whole amount of monomers, and the polymerization was started. The ammonium persulfate aqueous solution was added at such an amount that the amount of ammonium persulfate was 0.5% by mass after 4 hours, 1.0% by mass after 20 and 26 hours, and 1.5% by mass after 45 and 51 hours to the whole amount of monomers, and the mixture was reacted for 68 hours.

FIG. 3 shows melting curves of a double strand obtained from the reaction mixtures 1, 2 and 5, and FIG. 4 shows melting curves of a double strand obtained from the reaction mixtures 3, 4 and 6. As shown in FIG. 3, a Tm value of a double strand between the standard nucleic acid and the complementary strand was 37.00°C in the absence of the polymer (agent for raising a Tm of a double-stranded nucleic acid). On the other hand, a Tm value of a double strand between the standard nucleic acid and the complementary strand was 69.85°C in the presence of the polymer (agent for raising a Tm of a double-stranded nucleic acid), and a 32.85°C-rise in the Tm value was observed.

Further, as shown in FIG. 4, a Tm value of a double strand between the mutation strand and the complementary strand was 34.25°C in the absence of the polymer (agent for raising a Tm of a double-stranded nucleic acid). On the other hand, a Tm value of a double strand between the mutation strand and the complementary strand was 56.05°C in the presence of the polymer (agent for raising a Tm of a double-stranded nucleic acid), and a 21.80-rise in the Tm value was observed.

As a result, a difference of 11.05°C was observed between the rise in the Tm value of a double strand between the standard nucleic acid and the complementary strand and the rise in the Tm value of a double strand between the mutation strand and the complementary strand in the presence of the polymer (agent for raising a Tm of a double-stranded nucleic acid).

### [Example 3]

A melting curve of a double strand was obtained from each reaction mixture in a similar manner to Example 1 except that a diallylmethylamine/maleic acid copolymer obtained by copolymerizing diallylmethylamine (monomer 1) and maleic acid (monomer 2) under the following polymerization condition 3 and having a molar ratio of a constituent unit 1 derived from the monomer 1 and a constituent unit 2 derived from the monomer 2 of 1 : 1 was used as the agent for raising a Tm of a double-stranded nucleic acid, and then the Tm rising range (standard ΔTm) between the standard nucleic acid and the complementary strand and Tm rising range (sample ΔTm) between the mutation strand and the complementary strand due to the presence of a polymer (agent for raising a Tm of a double-stranded nucleic acid) were determined, and the difference (ΔΔTm) between them was calculated.

Polymerization condition 3: Into a 20-L four-neck flask equipped with a thermometer, a stirrer, and a cooling pipe, 1.86 kg (19.0 mol) of maleic anhydride and 0.34 kg of distilled water were charged, and 2.11 kg (19.0 mol) of diallylmethylamine (DAMA) was added dropwise under cooling. After that, the internal temperature was raised to 50°C. 28.5% by mass of ammonium persulfate aqueous solution was added at such an amount that the amount of ammonium persulfate in the aqueous solution was 0.5% by mass to the whole amount of monomers, and the polymerization was started. After 3, 21, and 25 hours, the 28.5% by mass of ammonium persulfate aqueous solution was added at such an amount that the amount of ammonium persulfate in the aqueous solution was 1.0% by mass to the whole amount of monomers, and the mixture was further reacted overnight.

FIG. 5 shows melting curves of a double strand obtained from the reaction mixtures 1, 2 and 5, and FIG. 6 shows melting curves of a double strand obtained from the reaction mixtures 3, 4 and 6. As shown in FIG. 5, a Tm value of a double strand between the standard nucleic acid and the complementary strand was 37.00°C in the absence of the polymer (agent for raising a Tm of a double-stranded nucleic acid). On the other hand, a Tm value of a double strand between the standard nucleic acid and the complementary strand was 65.05°C in the presence of the polymer (agent for raising a Tm of a double-stranded nucleic acid), and a 28.05°C-rise in the Tm value was observed.

Further, as shown in FIG. 6, a Tm value of a double strand between the mutation strand and the complementary strand was 34.25°C in the absence of the polymer (agent for raising a Tm of a double-stranded nucleic acid). On the other hand, a Tm value of a double strand between the mutation strand and the complementary strand was 50.95°C in the presence of the polymer (agent for raising a Tm of a double-stranded nucleic acid), and a 16.70-rise in the Tm value was observed.

As a result, a difference of 11.35°C was observed between the rise in the Tm value of a double strand between the standard nucleic acid and the complementary strand and the rise in the Tm value of a double strand between the mutation strand and the complementary strand in the presence of the polymer (agent for raising a Tm of a double-stranded nucleic acid).

### [Example 4]

An oligo-oligo double strand melting test was performed except that a diallylamine hydrochloride/acrylamide/acrylic acid copolymer obtained by copolymerizing a diallylamine hydrochloride (monomer 1), acrylamide (monomer 2), and acrylic acid (monomer 3) under the following polymerization condition 4 and having a molar ratio of a constituent unit 1 derived from the monomer 1, a constituent unit 2 derived from the monomer 2, and a constituent unit 3 derived from the monomer 3 of 1 : 1 : 2 was used as the agent for raising a Tm of a double-stranded nucleic acid.

Polymerization condition 4: Into a 300-mL four-neck flask equipped with a thermometer, a stirrer, and a cooling pipe, 20.43 g (equivalent to 0.1 mol) of diallylamine hydrochloride (solid content concentration: 65.40% by mass) and 110.9 g of distilled water were charged, and heated to 65°C. 28.5% by mass of ammonium persulfate aqueous solution was added at such an amount that the amount of ammonium persulfate in an aqueous solution was 2.0 mol% to the whole amount of monomers, and after the lapse of 30 minutes, a solution obtained by mixing 7.11 g (equivalent to 0.1 mol) of acrylamide (solid content concentration: 97% by mass), 14.56 g (equivalent to 0.2 mol) of acrylic acid (solid content concentration: 99% by mass), and 21.15 g of distilled water was added dropwise over 3 hours, and the reaction was continued overnight.

FIG. 7 shows melting curves of a double strand obtained from the reaction mixtures 1, 2 and 5, and FIG. 8 shows melting curves of a double strand obtained from the reaction mixtures 3, 4 and 6. As shown in FIG. 7, a Tm value of a double strand between the standard nucleic acid and the complementary strand was 38.40°C in the absence of the polymer (agent for raising a Tm of a double-stranded nucleic acid). On the other hand, a Tm value of a double strand between the standard nucleic acid and the complementary strand was 67.68°C in the presence of the polymer (agent for raising a Tm of a double-stranded nucleic acid), and a 29.28°C-rise in the Tm value was observed.

Further, as shown in FIG. 8, a Tm value of a double strand between the mutation strand and the complementary strand was 31.64°C in the absence of the polymer (agent for raising a Tm of a double-stranded nucleic acid). On the other hand, a Tm value of a double strand between the mutation strand and the complementary strand was 53.39°C in the presence of the polymer (agent for raising a Tm of a double-stranded nucleic acid), and a 21.75-rise in the Tm value was observed.

As a result, a difference of 7.53°C was observed between the rise in the Tm value of a double strand between the standard nucleic acid and the complementary strand and the rise in the Tm value of a double strand between the mutation strand and the complementary strand in the presence of the polymer (agent for raising a Tm of a double-stranded nucleic acid).

### [Comparative Example 1]

A melting curve of a double strand was obtained from each reaction mixture in a similar manner to Example 1 except that an allylamine polymer (product name: PAA-03, manufactured by Nittobo Medical Co., Ltd.) having a weight average molecular weight (Mw) of 3000 obtained by GPC measurement was used as the agent for raising a Tm of a double-stranded nucleic acid, and then the Tm rising range (standard ΔTm) between the standard nucleic acid and the complementary strand and Tm rising range (sample ΔTm) between the mutation strand and the complementary strand due to the presence of a polymer (agent for raising a Tm of a double-stranded nucleic acid) were determined, and the difference (ΔΔTm) between them was calculated.

FIG. 9 shows melting curves of a double strand obtained from the reaction mixtures 1, 2 and 5, and FIG. 10 shows melting curves of a double strand obtained from the reaction mixtures 3, 4 and 6. As shown in FIGS. 9 and 10, in the presence of the polymer (agent for raising a Tm of a double-stranded nucleic acid), no clear peak was detected, and it was difficult to determine the Tm value.

### [Comparative Example 2]

A melting curve of a double strand was obtained from each reaction mixture in a similar manner to Example 1 except that an allylamine polymer (product name: PAA-08, manufactured by Nittobo Medical Co., Ltd.) having a weight average molecular weight (Mw) of 8000 obtained by GPC measurement was used as the agent for raising a Tm of a double-stranded nucleic acid, and then the Tm rising range (standard ΔTm) between the standard nucleic acid and the complementary strand and Tm rising range (sample ΔTm) between the mutation strand and the complementary strand due to the presence of a polymer (agent for raising a Tm of a double-stranded nucleic acid) were determined, and the difference (ΔΔTm) between them was calculated.

FIG. 11 shows melting curves of a double strand obtained from the reaction mixtures 1, 2 and 5, and FIG. 12 shows melting curves of a double strand obtained from the reaction mixtures 3, 4 and 6. As shown in FIGS. 11 and 12, in the presence of the polymer (agent for raising a Tm of a double-stranded nucleic acid), no clear peak was detected, and it was difficult to determine the Tm value.

### [Comparative Example 3]

A melting curve of a double strand was obtained from each reaction mixture in a similar manner to Example 1 except that an allylamine polymer (product name: PAA-15C, manufactured by Nittobo Medical Co., Ltd.) having a weight average molecular weight (Mw) of 15000 obtained by GPC measurement was used as the agent for raising a Tm of a double-stranded nucleic acid, and then the Tm rising range (standard ΔTm) between the standard nucleic acid and the complementary strand and Tm rising range (sample ΔTm) between the mutation strand and the complementary strand due to the presence of a polymer (agent for raising a Tm of a double-stranded nucleic acid) were determined, and the difference (ΔΔTm) between them was calculated.

FIG. 13 shows melting curves of a double-strand obtained from the reaction mixtures 1, 2 and 5, and FIG. 14 shows melting curves of a double strand obtained from the reaction mixtures 3, 4 and 6. As shown in FIGS. 13 and 14, in the presence of the polymer (agent for raising a Tm of a double-stranded nucleic acid), no clear peak was detected, and it was difficult to determine the Tm value.

### [Comparative Example 4]

An oligo-oligo double strand melting test was performed except that an allylamine hydrochloride/diallylamine hydrochloride copolymer obtained by copolymerizing an allylamine hydrochloride (monomer 1) and a diallylamine hydrochloride (monomer 2) under the following polymerization condition 5 and having a molar ratio of a constituent unit 1 derived from the monomer 1 and a constituent unit 2 derived from the monomer 2 of 1 : 1 and a weight average molecular weight (Mw) of 100000 obtained by GPC measurement was used as the agent for raising a Tm of a double-stranded nucleic acid.

Polymerization condition 5: Into a 1-L four-neck flask equipped with a thermometer, a stirrer, and a cooling pipe, 245.24 g (1.50 mol) of 57.22% by mass of allylamine hydrochloride and 307.31 g (1.50 mol) of 65.22% by mass of diallylamine hydrochloride were charged, and heated to 60°C. 30% by mass of V-50 (2,2'-azobis (2-methylpropionamidine) dihydrochloride) suspension was added at such an amount that the amount of V-50 in the suspension was 0.50% by mass to the whole amount of monomers, and the polymerization was started. After 3 hours, 30% by mass of V-50 (2,2'-azobis(2-methylpropionamidine)dihydrochloride) suspension was added at such an amount that the amount of V-50 in the suspension was 0.50% by mass, and after 24, 27, 48, 51, and 54 hours, at such an amount that the amount of V-50 in the suspension was 0.25% by mass, to the whole amount of monomers were added, and the mixture was further reacted overnight.

FIG. 15 shows melting curves of a double strand obtained from the reaction mixtures 1, 2 and 5, and FIG. 16 shows melting curves of a double strand obtained from the reaction mixtures 3, 4 and 6. As shown in FIGS. 15 and 16, in the presence of the polymer (agent for raising a Tm of a double-stranded nucleic acid), no clear peak was detected, and it was difficult to determine the Tm value.

### [Comparative Example 5]

An oligo-oligo double strand melting test was performed except that an allylamine hydrochloride/diallylamine hydrochloride copolymer obtained by copolymerizing an allylamine hydrochloride (monomer 1) and a diallylamine hydrochloride (monomer 2) under the following polymerization condition 6 and having a molar ratio of a constituent unit 1 derived from the monomer 1 and a constituent unit 2 derived from the monomer 2 of 4 : 1 and a weight average molecular weight (Mw) of 20000 obtained by GPC measurement was used as the agent for raising a Tm of a double-stranded nucleic acid.

Polymerization condition 6: Into a 1-L four-neck flask equipped with a thermometer, a stirrer, and a cooling pipe, 490.48 g (3.00 mol) of 57.22% by mass of allylamine hydrochloride and 153.66 g (0.75 mol) of 65.22% by mass of diallylamine hydrochloride were charged, and heated to 60°C. 30% by mass of V-50 (2,2'-azobis(2-methylpropionamidine)dihydrochloride) suspension was added at such an amount that the amount of V-50 in the suspension was 1.00% by mass to the whole amount of monomers, and the polymerization was started. After 24 hours, 30% by mass of V-50 (2,2'-azobis(2-methylpropionamidine)dihydrochloride) suspension was added at such an amount that the amount of V-50 in the suspension was 1.00% by mass, and after 48 hours, at such an amount that the amount of V-50 in the suspension was 0.50% by mass were added, and the mixture was further reacted overnight.

FIG. 17 shows melting curves of a double strand obtained from the reaction mixtures 1, 2 and 5, and FIG. 18 shows melting curves of a double strand obtained from the reaction mixtures 3, 4 and 6. As shown in FIGS. 17 and 18, in the presence of the polymer (agent for raising a Tm of a double-stranded nucleic acid), no clear peak was detected, and it was difficult to determine the Tm value.

### [Comparative Example 6]

A melting curve of a double strand was obtained from each reaction mixture in a similar manner to Example 1 except that 50 mol% acetylated polyallylamine having a weight average molecular weight (Mw) of 15000 obtained by GPC measurement obtained under the following reaction condition 1 was used, and then the Tm rising range (standard ΔTm) between the standard nucleic acid and the complementary strand and Tm rising range (sample ΔTm) between the mutation strand and the complementary strand due to the presence of a polymer (agent for raising a Tm of a double-stranded nucleic acid) were determined, and the difference (ΔΔTm) between them was calculated.

FIG. 19 shows melting curves of a double strand obtained from the reaction mixtures 1, 2 and 5, and FIG. 20 shows melting curves of a double strand obtained from the reaction mixtures 3, 4 and 6. As shown in FIGS. 19 and 20, in the presence of the polymer (agent for raising a Tm of a double-stranded nucleic acid), no clear peak was detected, and it was difficult to determine the Tm value.

Reaction condition 1: Into a 20-L four-neck flask equipped with a thermometer, a stirrer, and a cooling pipe, 17.00 kg (45.11 mol) of 15.15% by mass of allylamine polymer (product name: PAA-15C, manufactured by Nittobo Medical Co., Ltd. ) was charged. After that, 2.37 kg (22.55 mol) of acetic anhydride was added dropwise while cooling, and the mixture was reacted overnight at room temperature.

### [Comparative Example 7]

A melting curve of a double strand was obtained from each reaction mixture in a similar manner to Example 1 except that polyacrylamide having a weight average molecular weight (Mw) of 20000 obtained by GPC measurement obtained by polymerization under the following polymerization condition 7 was used, and then the Tm rising range (standard ΔTm) between the standard nucleic acid and the complementary strand and Tm rising range (sample ΔTm) between the mutation strand and the complementary strand due to the presence of a polymer (agent for raising a Tm of a double-stranded nucleic acid) were determined, and the difference (ΔΔTm) between them was calculated.

Polymerization condition 7: Into a 300-mL four-neck flask equipped with a thermometer, a stirrer, and a cooling pipe, 186.59 g of distilled water was charged, and heated to 60°C. 0.75 g of ammonium persulfate was added, and then a solution obtained by mixing 25.65 g (equivalent to 0.35 mol) of acrylamide (solid content concentration: 97% by mass) and 36.55 g of distilled water was added dropwise over 5 hours, and the reaction was continued overnight.

FIG. 21 shows melting curves of a double strand obtained from the reaction mixtures 1, 2 and 5, and FIG. 22 shows melting curves of a double strand obtained from the reaction mixtures 3, 4 and 6. As shown in FIG. 21, a Tm value of a double strand between the standard nucleic acid and the complementary strand was 38.4°C in the absence of the polymer (agent for raising a Tm of a double-stranded nucleic acid). On the other hand, a Tm value of a double strand between the standard nucleic acid and the complementary strand was 49.81°C in the presence of the polymer (agent for raising a Tm of a double-stranded nucleic acid), and a 11.41°C-rise in the Tm value was observed.

Further, as shown in FIG. 22, a Tm value of a double strand between the mutation strand and the complementary strand was 31.64°C in the absence of the polymer (agent for raising a Tm of a double-stranded nucleic acid). On the other hand, a Tm value of a double strand between the mutation strand and the complementary strand was 42.14°C in the presence of the polymer (agent for raising a Tm of a double-stranded nucleic acid), and a 10.50-rise in the Tm value was observed.

As a result, only a difference of 0.91°C was observed between the rise in the Tm value of a double strand between the standard nucleic acid and the complementary strand and the rise in the Tm value of a double strand between the mutation strand and the complementary strand in the presence of the polymer (agent for raising a Tm of a double-stranded nucleic acid).

The test results are summarized below.

**[Table 4]**

| | Example 1 | Example 2 | Example 3 | Example 4 |
|---|---|---|---|---|
| Agent for raising Tm of double-stranded nucleic acid | Dimethylamino propyl methacrylamide hydrochloride/ acrylamide/ acrylic acid copolymer | Diallylamine hydrochloride/ maleic acid copolymer | Diallylmethyla mine/maleic acid copolymer | Diallylamine hydrochloride/ acrylamide/ acrylic acid copolymer |
| Molar ratio of constituent unit | | | | |
| Cationic unit | 1 | 1 | 1 | 1 |
| Anionic unit | 2 | 1 | 1 | 2 |
| Nonionic unit | 1 | 0 | 0 | 1 |
| Cation/anion | 0.5 | 1 | 1 | 0.5 |
| Reference ΔTm (°C) | 30.30 | 32.85 | 28.05 | 29.28 |
| Sample ΔTm (°C) | 1 9.10 | 2 1.80 | 16.70 | 21.75 |
| Δ Δ Tm (°C) | 11.20 | 11.05 | 11.3 5 | 7. 5 3 |

**[Table 5]**

| | Comparative Example 1 | Comparative Example 2 | Comparative Example 3 | Comparative Example 4 |
|---|---|---|---|---|
| Agent for raising Tm of double-stranded nucleic acid | Polyallylamine (Mw: 3000) | Polyallylamine (Mw: 8000) | Polyallylamine (Mw: 15000) | Allylamine hydrochloride/ diallylamine hydrochloride copolymer (Mw: 100000) |
| Molar ratio of constituent unit | | | | |
| Cationic unit | 1 | 1 | 1 | 1 |
| Anionic unit | 0 | 0 | 0 | 0 |
| Nonionic unit | 0 | 0 | 0 | 0 |
| Cation/anion | - | - | - | - |
| Reference ΔTm (°C) | (Detection failure) | (Detection failure) | (Detection failure) | (Detection failure) |
| Sample ΔTm (°C) | (Detection failure) | (Detection failure) | (Detection failure) | (Detection failure) |
| Δ Tm (°C) | - | - | - | - |

**[Table 6]**

| | Comparative Example 5 | Comparative Example 6 | Comparative Example 7 | Reference Example 1 |
|---|---|---|---|---|
| Agent for raising Tm of double-stranded nucleic acid | Allylamine hydrochloride/ diallylamine hydrochloride copolymer (Mw: 20000) | 50 mol% Acetylated polyallylamine (Mw: 15000) | Polyacrylamide (Mw: 20000) | Magnesium chloride |
| Molar ratio of constituent unit | | | | - |
| Cationic unit | 1 | 1 | 0 | - |
| Anionic unit | 0 | 0 | 0 | - |
| Nonionic unit | 0 | 1 | 1 | - |
| Cation/anion | - | - | - | 1 |
| Reference ΔTm (°C) | (Detection failure) | (Detection failure) | 11. 4 | 16. 5 4 |
| Sample ΔTm (°C) | (Detection failure) | (Detection failure) | 10.5 | 14.9 4 |
| Δ Tm (°C) | - | - | 0.9 | 1.6 |

### [Example 5]

By using artificial oligo DNA that was partly constituted of BNA as the complementary strand with respect to a standard nucleic acid, a dimethylamino propyl methacrylamide hydrochloride/acrylamide/acrylic acid copolymer was evaluated for the detection of mismatching in a similar manner to Example 1.

### 1. Polymer aqueous solution (agent for raising Tm of double-stranded nucleic acid)

As the agent for raising a Tm of a double-stranded nucleic acid, a polymer aqueous solution containing 10.0% by mass of the dimethylamino propyl methacrylamide hydrochloride/acrylamide/acrylic acid copolymer used in Example 1 and having a pH of 7.0 was prepared.

### 2. Standard nucleic acid, nucleic acid consisting of complementary sequence, and nucleic acid having mutation

In order to evaluate whether or not mutation at one base with respect to the nucleotide sequence of a standard nucleic acid is detectable, the following oligo DNAs were used.

**[Table 7]**

| | | |
|---|---|---|
| Reference nucleic acid | 11-mer oligo DNA derived from part of Exon 2 of wild-type KRAS gene sense strand | GGTG**G**CGTAGG (SEQ ID NO: 1) |
| BNA consisting of sequence complementary to reference nucleic acid (may also be referred to as BNA complementary strand) | 11-mer oligo DNA of antisense strand consisting of sequence complementary to the sense strand oligo DNA. However, partly constituted of BNA being unnatural nucleotide. | CCTACG**C**CACC (SEQ ID NO: 4) |
| Nucleic acid having mutation in reference nucleic acid (may also be referred to as mutation strand) | 11-mer oligo DNA having mutation at one base with respect to the sense strand DNA oligomer | GGTG**A**CGTAGG (SEQ ID NO: 3) |

In a base sequence of each DNA oligomer in the table, a mismatch site is indicated with a bold letter. Further, the nucleotide containing the base indicated by the underline is BNA having the following structure:

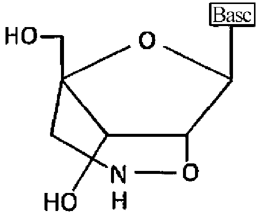

### 3. Preparation of reaction mixture

The following reaction mixtures 1 to 4 were prepared.

**[Table 8]**

| Reaction mixture 1 | |
|---|---|
| Components | Content |
| 500 µM of reference nucleic acid aqueous solution | 2.0*µ*L |
| 500 µM of BNA complementary strand aqueous solution | 2.0*µ*L |
| 25 µM of fluorescent dye solution (manufactured by Biotium, trade name: EvaGreen) | 1.0*µ*L |
| Nuclease-free water | 15.0*µ*L |

### Reaction mixture 2

| Components | Content |
|---|---|
| 500 µM of reference nucleic acid aqueous solution | 2.0*µ*L |
| 500 µM of BNA complementary strand aqueous solution | 2.0*µ*L |
| 25 µM of fluorescent dye solution (manufactured by Biotium, trade name: EvaGreen) | 1.0*µ*L |
| 10.0% by mass of polymer aqueous solution | 2.0*µ*L |
| Nuclease-free water | 13.0*µ*L |

### Reaction mixture 3

| Components | Content |
|---|---|
| 500 µM of mutation strand aqueous solution | 2.0*µ*L |
| 500 µM of BNA complementary strand aqueous solution | 2.0*µ*L |
| 25 µM of fluorescent dye solution (manufactured by Biotium, trade name: EvaGreen) | 1.0*µ*L |
| Nuclease-free water | 15.0*µ*L |

### Reaction mixture 4

| Components | Content |
|---|---|
| 500 µM of mutation strand aqueous solution | 2.0*µ*L |
| 500 µM of BNA complementary strand aqueous solution | 2.0*µ*L |
| 25 µM of fluorescent dye solution (manufactured by Biotium, trade name: EvaGreen) | 1.0*µ*L |
| 10.0% by mass of polymer aqueous solution | 2.0*µ*L |
| Nuclease-free water | 13.0*µ*L |

Concentration of the polymer (agent for raising a Tm of a double-stranded nucleic acid) in reaction mixtures 2 and 4 was 1.0% by mass.

### 4. Creation of melting curve of double strand

Each of the prepared reaction mixtures was set in StepOnePlus Real-Time PCR System (manufactured by Thermo Fisher Scientific), heated at 95°C for 15 seconds, and then kept at 10°C for 1 minute. Next, while performing fluorescence measurement every 0.3 seconds, the temperature was raised to 95°C to obtain a melting curve of a double-strand, and then held at 95°C for 15 seconds.

### [Test Results]

FIG. 23 shows melting curves of a double strand obtained from the reaction mixtures 1 and 2, and FIG. 24 shows melting curves of a double strand obtained from the reaction mixtures 3 and 4. As shown in FIG. 23, a Tm value of a double strand between the standard nucleic acid and the BNA complementary strand was 63.74°C in the absence of the polymer (agent for raising a Tm of a double-stranded nucleic acid). On the other hand, a Tm value of a double strand between the standard nucleic acid and the BNA complementary strand was 92.95°C in the presence of the polymer (agent for raising a Tm of a double-stranded nucleic acid), and a 29.21°C-rise in the Tm value was observed.

Further, as shown in FIG. 24, a Tm value of a double strand between the mutation strand and the BNA complementary strand was 62.40°C in the absence of the polymer (agent for raising a Tm of a double-stranded nucleic acid). On the other hand, a Tm value of a double strand between the mutation strand and the BNA complementary strand was 79.92°C in the presence of the polymer (agent for raising a Tm of a double-stranded nucleic acid), and a 17.52°C-rise in the Tm value was observed.

As a result, a difference of 11.69°C was observed between the rise in the Tm value of a double strand between the standard nucleic acid and the BNA complementary strand and the rise in the Tm value of a double strand between the mutation strand and the BNA complementary strand in the presence of the polymer (agent for raising Tm of a double-stranded nucleic acid).

### [Example 6]

A melting curve of a double strand was obtained from each reaction mixture in a similar manner to Example 5 except that a polymer aqueous solution containing 10.0% by mass of the diallylamine hydrochloride/maleic acid copolymer that was used in Example 2, and having a pH of 7.0 was used as the agent for raising a Tm of a double-stranded nucleic acid, and then the Tm rising range (standard ΔTm) between the standard nucleic acid and the BNA complementary strand and Tm rising range (sample ΔTm) between the mutation strand and the BNA complementary strand due to the presence of a polymer (agent for raising a Tm of a double-stranded nucleic acid) were determined, and the difference (ΔΔTm) between them was calculated.

FIG. 25 shows melting curves of a double strand obtained from the reaction mixtures 1 and 2, and FIG. 26 shows melting curves of a double strand obtained from the reaction mixtures 3 and 4, respectively. As shown in FIG. 25, a Tm value of a double strand between the standard nucleic acid and the BNA complementary strand was 63.74°C in the absence of the polymer (agent for raising a Tm of a double-stranded nucleic acid). On the other hand, a Tm value of a double strand between the standard nucleic acid and the BNA complementary strand was 92.95°C in the presence of the polymer (agent for raising a Tm of a double-stranded nucleic acid), and a 29.21°C-rise in the Tm value was observed.

Further, as shown in FIG. 26, a Tm value of a double strand between the mutation strand and the BNA complementary strand was 62.40°C in the absence of the polymer (agent for raising a Tm of a double-stranded nucleic acid). On the other hand, a Tm value of a double strand between the mutation strand and the BNA complementary strand was 81.10°C in the presence of the polymer (agent for raising a Tm of a double-stranded nucleic acid), and an 18.60°C-rise in the Tm value was observed.

As a result, a difference of 10.61°C was observed between the rise in the Tm value of a double strand between the standard nucleic acid and the BNA complementary strand and the rise in the Tm value of a double strand between the mutation strand and the BNA complementary strand in the presence of the polymer (agent for raising Tm of a double-stranded nucleic acid).

The test results of Examples 5 and 6 are summarized below.

**[Table 9]**

| | Example 5 | Example 6 |
|---|---|---|
| Agent for raising Tm of double-stranded nucleic acid | Dimethylamino propyl methacrylamide hydrochloride/acrylamide/ acrylic acid copolymer | Diallylamine hydrochloride/ maleic acid copolymer |
| Molar ratio of constituent unit | | |
| Cationic unit | 1 | 1 |
| Anionic unit | 2 | 1 |
| Nonionic unit | 1 | 0 |
| Cation/anion | 0.5 | 1 |
| BNA complementary strand | ○ | ○ |
| Reference ΔTm (°C) | 29. 21 | 29.21 |
| Sample ΔTm (°C) | 17.52 | 18.60 |
| Δ Tm (°C) | 11.69 | 10.61 |

## Claims

1. Use of an agent for raising a melting temperature of a double-stranded nucleic acid, comprising
an amphoteric copolymer, wherein the amphoteric copolymer comprises:
at least one kind of cationic constituent unit (1) selected from the group consisting of
a constituent unit (1-1) having a structure represented by general formula (I-a) or general formula (I-b), or a structure of an acid addition salt thereof: 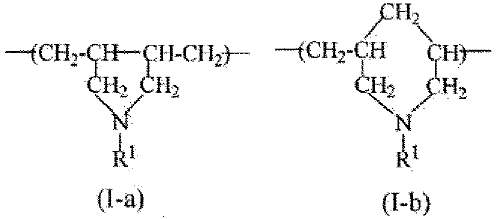
wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
a constituent unit (1-4) having a structure represented by general formula (1-f), or a structure of an acid addition salt thereof: 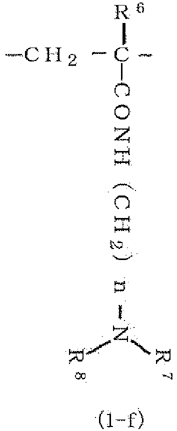
wherein R⁶ represents a hydrogen atom or a methyl group, R⁷ and R⁸ each independently represent an alkyl group having 1 to 4 carbon atoms, and n is an integer of 2 to 4; and
at least one kind of anionic constituent unit (2) selected from the group consisting of
a constituent unit (2-1) having a structure represented by general formula (II-a): 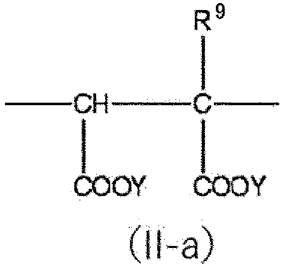
wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
a constituent unit (2-4) having a structure represented by general formula (II-d): 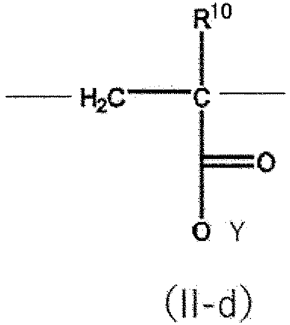
wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

2. The use of an agent for raising a melting temperature of a double-stranded nucleic acid according to claim 1, wherein the amphoteric copolymer further comprises a nonionic constituent unit (3).

3. The use of an agent for raising the melting temperature according to claim 2, wherein the nonionic constituent unit (3) is a constituent unit derived from a methacrylamide-based monomer, or an acrylamide-based monomer.

4. A method for detecting nucleic acid mismatching between a sample nucleic acid and a probe nucleic acid, comprising the steps of:
(1) measuring melting temperatures of a double-stranded nucleic acid between the probe nucleic acid and a nucleic acid having a base sequence complementary to the probe nucleic acid with and without adding an agent for raising a melting temperature of a double-stranded nucleic acid, and determining a rise value between the melting temperatures of the probe nucleic acid and the nucleic acid having a base sequence complementary to the probe nucleic acid due to the presence of the amphoteric copolymer (standard ΔTm);
(2) measuring melting temperatures of a double-stranded nucleic acid between the sample nucleic acid and the probe nucleic acid with and without adding an agent for raising a melting temperature of a double-stranded nucleic acid, and determining a rise value between the melting temperatures of the double-stranded nucleic acid of the sample nucleic acid and the probe nucleic acid due to the presence of the amphoteric copolymer (sample ΔTm); and
(3) determining the presence or absence of nucleic acid mismatching based on a value (ΔΔTm) obtained by subtracting the sample ΔTm from the standard ΔTm,
wherein the agent for raising a melting temperature of a double-stranded nucleic acid comprises:
an amphoteric copolymer, wherein the amphoteric copolymer comprises:
at least one kind of cationic constituent unit (1) selected from the group consisting of
a constituent unit (1-1) having a structure represented by general formula (I-a) or general formula (I-b), or a structure of an acid addition salt thereof: 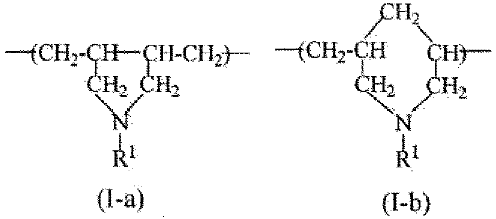
wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
a constituent unit (1-4) having a structure represented by general formula (1-f), or a structure of an acid addition salt thereof: 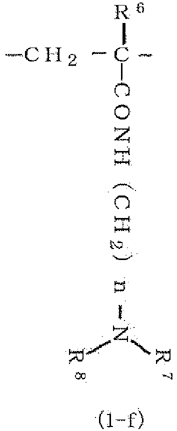
wherein R⁶ represents a hydrogen atom or a methyl group, R⁷ and R⁸ each independently represent an alkyl group having 1 to 4 carbon atoms, and n is an integer of 2 to 4; and
at least one kind of anionic constituent unit (2) selected from the group consisting of
a constituent unit (2-1) having a structure represented by general formula (II-a): 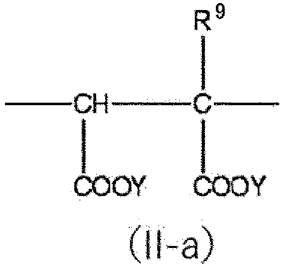
wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
a constituent unit (2-4) having a structure represented by general formula (II-d): 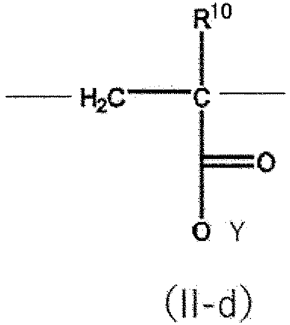
wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

5. The method for detecting nucleic acid mismatching according to claim 4, wherein it is determined that the nucleic acid mismatching is present when the ΔΔTm is 5.0°C or more.

6. The method for detecting nucleic acid mismatching according to claim 4 or 5, wherein the amphoteric copolymer further comprises a nonionic constituent unit (3).

7. The method for detecting nucleic acid mismatching according to claim 6, wherein the nonionic constituent unit (3) is a constituent unit derived from a methacrylamide-based monomer, or an acrylamide-based monomer.

8. A kit for:
(i) detecting nucleic acid mismatching between a sample nucleic acid and a probe nucleic acid, comprising the probe nucleic acid and a nucleic acid having a base sequence complementary to the probe nucleic acid; or
(ii) detecting mutation between a sample nucleic acid and a standard nucleic acid, comprising the standard nucleic acid and a nucleic acid having a base sequence complementary to the standard nucleic acid;
wherein the kit comprises
an agent for raising a melting temperature of a double-stranded nucleic acid,
wherein the agent for raising a melting temperature of a double-stranded nucleic acid comprises:
an amphoteric copolymer, wherein the amphoteric copolymer comprises:
at least one kind of cationic constituent unit (1) selected from the group consisting of
a constituent unit (1-1) having a structure represented by general formula (I-a) or general formula (I-b), or a structure of an acid addition salt thereof: 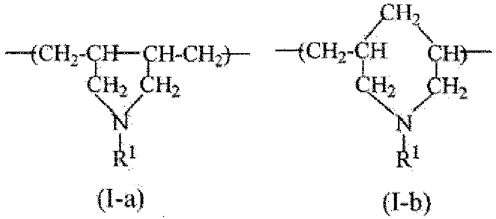
wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
a constituent unit (1-4) having a structure represented by general formula (1-f), or a structure of an acid addition salt thereof: 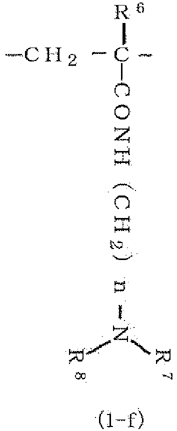
wherein R⁶ represents a hydrogen atom or a methyl group, R⁷ and R⁸ each independently represent an alkyl group having 1 to 4 carbon atoms, and n is an integer of 2 to 4; and
at least one kind of anionic constituent unit (2) selected from the group consisting of
a constituent unit (2-1) having a structure represented by general formula (II-a): 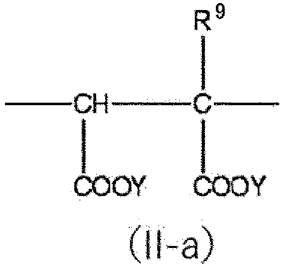
wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
a constituent unit (2-4) having a structure represented by general formula (II-d): 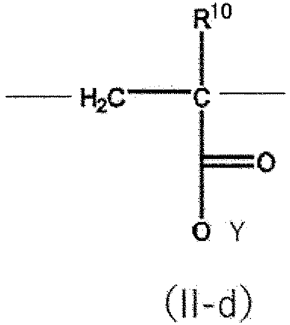
wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

9. The kit for detecting nucleic acid mismatching according to claim 8, wherein the amphoteric copolymer further comprises a nonionic constituent unit (3).

10. The kit for detecting nucleic acid mismatching according to claim 9, wherein the nonionic constituent unit (3) is a constituent unit derived from a methacrylamide-based monomer, or an acrylamide-based monomer.

11. A method for detecting mutation with respect to a standard nucleic acid in a sample nucleic acid, comprising the steps of:
(1) measuring melting temperatures of a double-stranded nucleic acid between the standard nucleic acid and a nucleic acid having a base sequence complementary to the standard nucleic acid with and without adding an agent for raising a melting temperature of a double-stranded nucleic acid, and determining a rise value (standard ΔTm) between the melting temperatures of the double-stranded nucleic acid between the standard nucleic acid and the nucleic acid having the base sequence complementary to the standard nucleic acid due to the presence of the amphoteric copolymer;
(2) measuring melting temperatures of a double-stranded nucleic acid of the sample nucleic acid and the nucleic acid having a base sequence complementary to the standard nucleic acid with and without adding an agent for raising a melting temperature of a double-stranded nucleic acid, and determining a rise value (sample ΔTm) between the melting temperatures of the double-stranded nucleic acid between the sample nucleic acid and the nucleic acid having the base sequence complementary to the standard nucleic acid due to the presence of the amphoteric copolymer; and
(3) determining the presence or absence of mutation based on a value (ΔΔTm) obtained by subtracting the sample ΔTm from the standard ΔTm,
wherein the agent for raising a melting temperature of a double-stranded nucleic acid comprises:
an amphoteric copolymer, wherein the amphoteric copolymer comprises:
at least one kind of cationic constituent unit (1) selected from the group consisting of
a constituent unit (1-1) having a structure represented by general formula (I-a) or general formula (I-b), or a structure of an acid addition salt thereof: 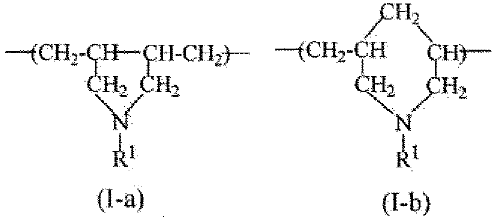
wherein R¹ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and
a constituent unit (1-4) having a structure represented by general formula (1-f), or a structure of an acid addition salt thereof: 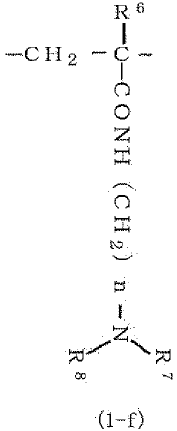
wherein R⁶ represents a hydrogen atom or a methyl group, R⁷ and R⁸ each independently represent an alkyl group having 1 to 4 carbon atoms, and n is an integer of 2 to 4; and
at least one kind of anionic constituent unit (2) selected from the group consisting of
a constituent unit (2-1) having a structure represented by general formula (II-a): 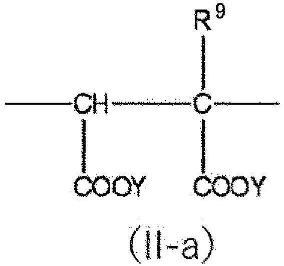
wherein R⁹ is a hydrogen or a methyl group, Y represents a hydrogen, Na, K, NH₄, 1/2Ca, 1/2Mg, 1/2Fe, 1/3Al, or 1/3Fe independently for each carboxy group to be bonded, and
a constituent unit (2-4) having a structure represented by general formula (II-d): 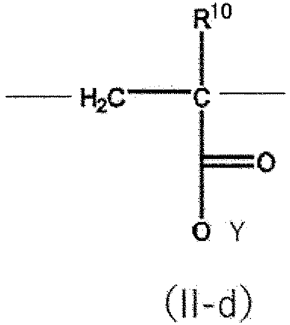
wherein R¹⁰ is a hydrogen or a methyl group, Y represents a hydrogen, Na, K, NH₄, 1/2Ca, 1/2Mg, 1/2Fe, 1/3Al, or 1/3Fe independently for each carboxy group to be bonded.

12. The method for detecting mutation according to claim 11, wherein it is determined that mutation is present when the ΔΔTm is 5.0°C or more.

13. The method for detecting mutation according to claim 11 or 12, wherein the amphoteric copolymer further comprises a nonionic constituent unit (3), preferably wherein the nonionic constituent unit (3) is a constituent unit derived from a methacrylamide-based monomer or an acrylamide-based monomer.

## Patentansprüche

1. Verwendung eines Agens zur Erhöhung einer Schmelztemperatur einer doppelsträngigen Nukleinsäure, umfassend
ein amphoteres Copolymer, wobei das amphotere Copolymer umfasst:
mindestens eine Art einer kationischen Struktureinheit ("constituent unit") (1), ausgewählt aus der Gruppe bestehend aus
einer Struktureinheit (1-1) mit einer Struktur, die durch die allgemeine Formel (I-a) oder die allgemeine Formel (I-b) dargestellt wird, oder einer Struktur eines Säureadditionssalzes davon: 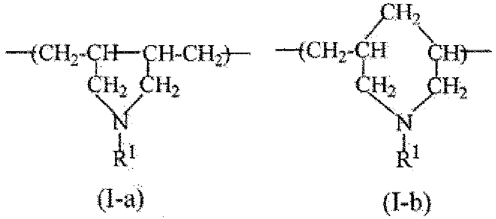
wobei R¹ ein Wasserstoffatom, eine Alkylgruppe mit 1 bis 10 Kohlenstoffatomen, die eine Hydroxylgruppe aufweisen können, eine Cycloalkylgruppe mit 5 bis 10 Kohlenstoffatomen oder eine Aralkylgruppe mit 7 bis 10 Kohlenstoffatomen darstellt, und
eine Struktureinheit (1-4) mit einer Struktur, die durch die allgemeine Formel (1-f) dargestellt wird, oder eine Struktur eines Säureadditionssalzes davon: 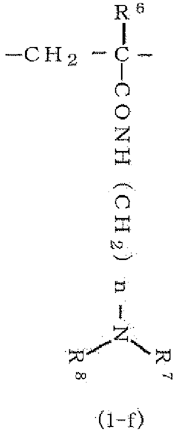
wobei R⁶ ein Wasserstoffatom oder eine Methylgruppe darstellt, R⁷ und R⁸ jeweils unabhängig voneinander eine Alkylgruppe mit 1 bis 4 Kohlenstoffatomen darstellen und n eine ganze Zahl von 2 bis 4 ist; und
mindestens eine Art einer anionischen Struktureinheit (2), ausgewählt aus der Gruppe bestehend aus einer Struktureinheit (2-1) mit einer Struktur, die durch die allgemeine Formel (II-a) dargestellt wird: 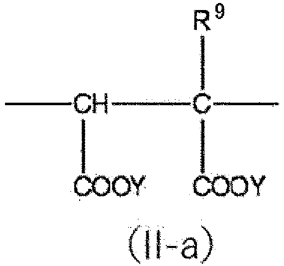
wobei R⁹ ein Wasserstoff oder eine Methylgruppe ist, Y ein Wasserstoff, Na, K, NH₄, 1/2Ca, 1/2Mg, 1/2Fe, 1/3Al oder 1/3Fe unabhängig für jede zu bindende Carboxygruppe darstellt, und
eine Struktureinheit (2-4) mit einer Struktur, die durch die allgemeine Formel (II-d) dargestellt wird: 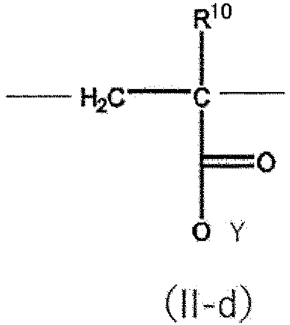
wobei R¹⁰ ein Wasserstoff oder eine Methylgruppe ist, Y ein Wasserstoff, Na, K, NH₄, 1/2Ca, 1/2Mg, 1/2Fe, 1/3Al oder 1/3Fe unabhängig für jede zu bindende Carboxygruppe darstellt.

2. Verwendung eines Agens zur Erhöhung einer Schmelztemperatur einer doppelsträngigen Nukleinsäure gemäß Anspruch 1, wobei das amphotere Copolymer ferner eine nichtionische Struktureinheit (3) umfasst.

3. Verwendung eines Agens zur Erhöhung der Schmelztemperatur gemäß Anspruch 2, wobei die nichtionische Struktureinheit (3) eine Struktureinheit ist, die von einem Methacrylamid-basierten Monomer oder einem Acrylamid-basierten Monomer abgeleitet ist.

4. Verfahren zum Nachweis von Fehlpaarung von Nukleinsäuren zwischen einer Nukleinsäure-Probe und einer Nukleinsäure-Sonde, umfassend die Schritte von:
(1) Messen der Schmelztemperaturen einer doppelsträngigen Nukleinsäure zwischen der Nukleinsäure-Sonde und einer Nukleinsäure mit einer zur Nukleinsäure-Sonde komplementären Basensequenz mit und ohne Zugabe eines Agens zur Erhöhung der Schmelztemperatur einer doppelsträngigen Nukleinsäure, und Bestimmen eines Anstiegswerts zwischen den Schmelztemperaturen der Nukleinsäure-Sonde und der Nukleinsäure, die eine zur Nukleinsäure-Sonde komplementären Basensequenz hat, aufgrund der Anwesenheit des amphoteren Copolymers (Standard-ΔTm);
(2) Messen der Schmelztemperaturen einer doppelsträngigen Nukleinsäure zwischen der Nukleinsäure-Probe und der Nukleinsäure-Sonde mit und ohne Zugabe eines Agens zur Erhöhung einer Schmelztemperatur einer doppelsträngigen Nukleinsäure und Bestimmen eines Anstiegswerts zwischen den Schmelztemperaturen der doppelsträngigen Nukleinsäure der Nukleinsäure-Probe und der Nukleinsäure-Sonde aufgrund des Vorhandenseins des amphoteren Copolymers (Proben-ΔTm); und
(3) Bestimmen des Vorhandenseins oder Fehlens einer Fehlpaarung von Nukleinsäuren basierend auf einem Wert (ΔΔTm), der durch Subtraktion des Proben-ΔTm vom Standard-ΔTm erhalten wird,
wobei das Agens zum Erhöhen der Schmelztemperatur einer doppelsträngigen Nukleinsäure umfasst:
ein amphoteres Copolymer, wobei das amphotere Copolymer umfasst:
mindestens eine Art einer kationischen Struktureinheit (1), ausgewählt aus der Gruppe bestehend aus einer Struktureinheit (1-1) mit einer Struktur, die durch die allgemeine Formel (I-a) oder die allgemeine Formel (I-b) dargestellt wird, oder einer Struktur eines Säureadditionssalzes davon: 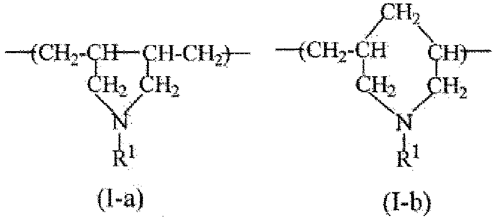
wobei R¹ ein Wasserstoffatom, eine Alkylgruppe mit 1 bis 10 Kohlenstoffatomen, die eine Hydroxylgruppe aufweisen können, eine Cycloalkylgruppe mit 5 bis 10 Kohlenstoffatomen oder eine Aralkylgruppe mit 7 bis 10 Kohlenstoffatomen darstellt, und
eine Struktureinheit (1-4) mit einer Struktur, die durch die allgemeine Formel (1-f) dargestellt wird, oder eine Struktur eines Säureadditionssalzes davon: 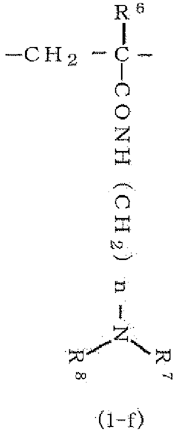
wobei R6 ein Wasserstoffatom oder eine Methylgruppe darstellt, R⁷ und R⁸ jeweils unabhängig voneinander eine Alkylgruppe mit 1 bis 4 Kohlenstoffatomen darstellen und n eine ganze Zahl von 2 bis 4 ist; und
mindestens eine Art einer anionischen Struktureinheit (2), ausgewählt aus der Gruppe bestehend aus einer Struktureinheit (2-1) mit einer Struktur, die durch die allgemeine Formel (II-a) dargestellt wird: 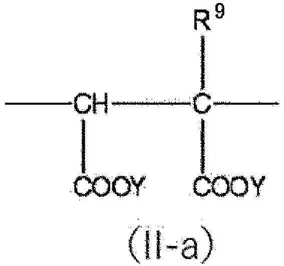
wobei R⁹ ein Wasserstoff oder eine Methylgruppe ist, Y ein Wasserstoff, Na, K, NH₄, 1/2Ca, 1/2Mg, 1/2Fe, 1/3Al oder 1/3Fe unabhängig für jede zu bindende Carboxygruppe darstellt, und
eine Struktureinheit (2-4) mit einer Struktur, die durch die allgemeine Formel (II-d) dargestellt wird: 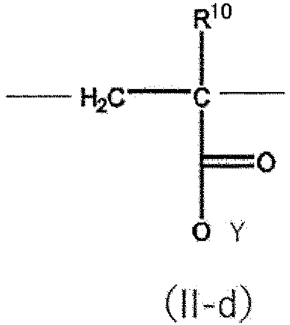
wobei R¹⁰ ein Wasserstoff oder eine Methylgruppe ist, Y ein Wasserstoff, Na, K, NH₄, 1/2Ca, 1/2Mg, 1/2Fe, 1/3Al oder 1/3Fe unabhängig für jede zu bindende Carboxygruppe darstellt.

5. Verfahren zum Nachweis einer Fehlpaarung von Nukleinsäuren gemäß Anspruch 4, wobei festgestellt wird, dass eine Fehlpaarung von Nukleinsäuren vorliegt, wenn ΔΔTm 5,0 °C oder mehr beträgt.

6. Verfahren zum Nachweis einer Fehlpaarung von Nukleinsäuren gemäß Anspruch 4 oder 5, wobei das amphotere Copolymer ferner eine nichtionische Struktureinheit (3) umfasst.

7. Verfahren zum Nachweis einer Fehlpaarung von Nukleinsäuren gemäß Anspruch 6, wobei die nichtionische Struktureinheit (3) eine Struktureinheit ist, die von einem Methacrylamid-basierten Monomer oder einem Acrylamid-basierten Monomer abgeleitet ist.

8. Ein Kit zum:
(i) Nachweisen einer Fehlpaarung von Nukleinsäuren zwischen einer Nukleinsäure-Probe und einer Nukleinsäure-Sonde, umfassend die Nukleinsäure-Sonde und eine Nukleinsäure mit einer zur Nukleinsäure-Sonde komplementären Basensequenz; oder
(ii) Nachweisen einer Mutation zwischen einer Nukleinsäure-Probe und einer Standard-Nukleinsäure, umfassend die Standard-Nukleinsäure und eine Nukleinsäure mit einer zur Standard-Nukleinsäure komplementären Basensequenz;
wobei das Kit umfasst
ein Agens zur Erhöhung einer Schmelztemperatur einer doppelsträngigen Nukleinsäure,
wobei das Agens zur Erhöhung einer Schmelztemperatur einer doppelsträngigen Nukleinsäure umfasst:
ein amphoteres Copolymer, wobei das amphotere Copolymer umfasst:
mindestens eine Art einer kationischen Struktureinheit (1), ausgewählt aus der Gruppe bestehend aus einer Struktureinheit (1-1) mit einer Struktur, die durch die allgemeine Formel (I-a) oder die allgemeine Formel (I-b) dargestellt wird, oder einer Struktur eines Säureadditionssalzes davon: 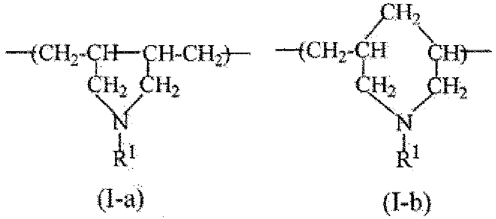
wobei R¹ ein Wasserstoffatom, eine Alkylgruppe mit 1 bis 10 Kohlenstoffatomen, die eine Hydroxylgruppe aufweisen können, eine Cycloalkylgruppe mit 5 bis 10 Kohlenstoffatomen oder eine Aralkylgruppe mit 7 bis 10 Kohlenstoffatomen darstellt, und
eine Struktureinheit (1-4) mit einer Struktur, die durch die allgemeine Formel (1-f) dargestellt wird, oder eine Struktur eines Säureadditionssalzes davon: 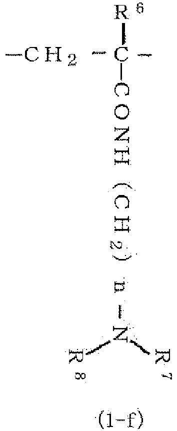
wobei R⁶ ein Wasserstoffatom oder eine Methylgruppe darstellt, R⁷ und R⁸ jeweils unabhängig voneinander eine Alkylgruppe mit 1 bis 4 Kohlenstoffatomen darstellen und n eine ganze Zahl von 2 bis 4 ist; und
mindestens eine Art einer anionischen Struktureinheit (2), ausgewählt aus der Gruppe bestehend aus
einer Struktureinheit (2-1) mit einer Struktur, die durch die allgemeine Formel (II-a) dargestellt wird: 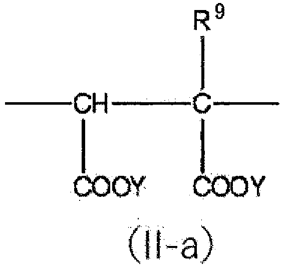
wobei R⁹ ein Wasserstoff oder eine Methylgruppe ist, Y ein Wasserstoff, Na, K, NH₄, 1/2Ca, 1/2Mg, 1/2Fe, 1/3Al oder 1/3Fe unabhängig für jede zu bindende Carboxygruppe darstellt, und
eine Struktureinheit (2-4) mit einer Struktur, die durch die allgemeine Formel (II-d) dargestellt wird: 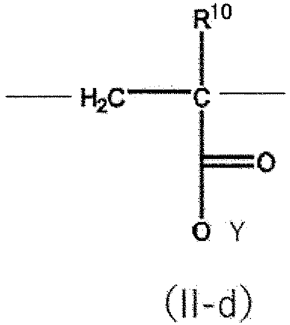
wobei R¹⁰ ein Wasserstoff oder eine Methylgruppe ist, Y ein Wasserstoff, Na, K, NH₄, 1/2Ca, 1/2Mg, 1/2Fe, 1/3Al oder 1/3Fe unabhängig für jede zu bindende Carboxygruppe darstellt.

9. Kit zum Nachweis einer Fehlpaarung von Nukleinsäuren gemäß Anspruch 8, wobei das amphotere Copolymer ferner eine nichtionische Struktureinheit (3) umfasst.

10. Kit zum Nachweis einer Fehlpaarung von Nukleinsäuren gemäß Anspruch 9, wobei die nichtionische Struktureinheit (3) eine Struktureinheit ist, die von einem Methacrylamid-basierten Monomer oder einem Acrylamid-basierten Monomer abgeleitet ist.

11. Verfahren zum Nachweisen einer Mutation in Bezug auf eine Standard-Nukleinsäure in einer Nukleinsäure-Probe, umfassend die Schritte von:
(1) Messen der Schmelztemperaturen einer doppelsträngigen Nukleinsäure zwischen der Standard-Nukleinsäure und einer Nukleinsäure mit einer zur Standard-Nukleinsäure komplementären Basensequenz mit und ohne Zugabe eines Agens zur Erhöhung einer Schmelztemperatur einer doppelsträngigen Nukleinsäure, und Bestimmen eines Anstiegswerts (Standard-ΔTm) zwischen den Schmelztemperaturen der doppelsträngigen Nukleinsäure zwischen der Standard-Nukleinsäure und der Nukleinsäure mit der zur Standard-Nukleinsäure komplementären Basensequenz aufgrund des Vorhandenseins des amphoteren Copolymers;
(2) Messen der Schmelztemperaturen einer doppelsträngigen Nukleinsäure der Nukleinsäure-Probe und der Nukleinsäure mit einer zur Standard-Nukleinsäure komplementären Basensequenz mit und ohne Zugabe eines Agens zur Erhöhung einer Schmelztemperatur einer doppelsträngigen Nukleinsäure, und Bestimmen eines Anstiegswerts (Proben-ΔTm) zwischen den Schmelztemperaturen der doppelsträngigen Nukleinsäure zwischen der Nukleinsäure-Probe und der Nukleinsäure mit der zur Standard-Nukleinsäure komplementären Basensequenz aufgrund des Vorhandenseins des amphoteren Copolymers; und
(3) Bestimmen des Vorhandenseins oder Fehlens einer Mutation basierend auf einem Wert (ΔΔTm), der durch Subtrahieren des Proben-ΔTm vom Standard-ΔTm erhalten wird,
wobei das Agens zum Erhöhen einer Schmelztemperatur einer doppelsträngigen Nukleinsäure umfasst:
ein amphoteres Copolymer, wobei das amphotere Copolymer umfasst:
mindestens eine Art einer kationischen Struktureinheit (1), ausgewählt aus der Gruppe bestehend aus einer Struktureinheit (1-1) mit einer Struktur, die durch die allgemeine Formel (I-a) oder die allgemeine Formel (I-b) dargestellt wird, oder einer Struktur eines Säureadditionssalzes davon: 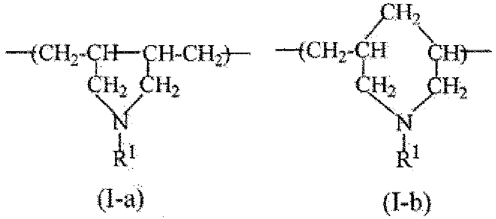
wobei R¹ ein Wasserstoffatom, eine Alkylgruppe mit 1 bis 10 Kohlenstoffatomen, die eine Hydroxylgruppe aufweisen können, eine Cycloalkylgruppe mit 5 bis 10 Kohlenstoffatomen oder eine Aralkylgruppe mit 7 bis 10 Kohlenstoffatomen darstellt, und
eine Struktureinheit (1-4) mit einer Struktur, die durch die allgemeine Formel (1-f) dargestellt wird, oder eine Struktur eines Säureadditionssalzes davon: 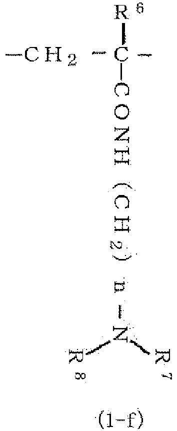
wobei R⁶ ein Wasserstoffatom oder eine Methylgruppe darstellt, R⁷ und R⁸ jeweils unabhängig voneinander eine Alkylgruppe mit 1 bis 4 Kohlenstoffatomen darstellen und n eine ganze Zahl von 2 bis 4 ist; und
mindestens eine Art einer anionischen Struktureinheit (2), ausgewählt aus der Gruppe bestehend aus einer Struktureinheit (2-1) mit einer Struktur, die durch die allgemeine Formel (II-a) dargestellt wird: 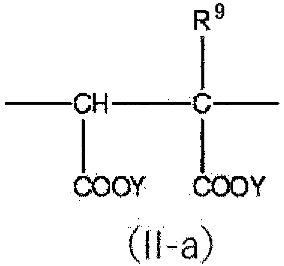
wobei R⁹ ein Wasserstoff oder eine Methylgruppe ist, Y ein Wasserstoff, Na, K, NH₄, 1/2Ca, 1/2Mg, 1/2Fe, 1/3Al oder 1/3Fe unabhängig für jede zu bindende Carboxygruppe darstellt, und
eine Struktureinheit (2-4) mit einer Struktur, die durch die allgemeine Formel (II-d) dargestellt wird: 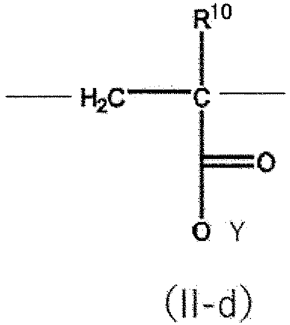
wobei R¹⁰ ein Wasserstoff oder eine Methylgruppe ist, Y ein Wasserstoff, Na, K, NH₄, 1/2Ca, 1/2Mg, 1/2Fe, 1/3Al oder 1/3Fe unabhängig für jede zu bindende Carboxygruppe darstellt.

12. Verfahren zum Nachweis einer Mutation gemäß Anspruch 11, wobei festgestellt wird, dass eine Mutation vorliegt, wenn ΔΔTm 5,0 °C oder mehr beträgt.

13. Verfahren zum Nachweis einer Mutation gemäß Anspruch 11 oder 12, wobei das amphotere Copolymer ferner eine nichtionische Struktureinheit (3) umfasst, vorzugsweise wobei die nichtionische Struktureinheit (3) eine Struktureinheit ist, die von einem Methacrylamid-basierten Monomer oder einem Acrylamid-basierten Monomer abgeleitet ist.

## Revendications

1. Utilisation d'un agent destiné à augmenter une température de fusion d'un acide nucléique double brin, comprenant
un copolymère amphotère, dans lequel le copolymère amphotère comprend :
au moins un type de motif constitutif cationique (1) sélectionné à partir du groupe constitué
d'un motif constitutif (1-1) présentant une structure représentée par la formule générale (I-a) ou la formule générale (I-b) ou une structure d'un sel d'addition d'acide de celui-ci : 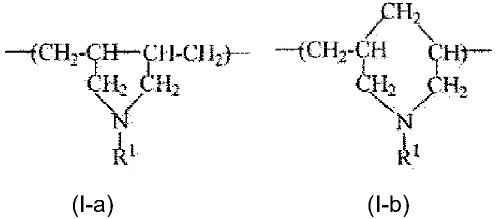
dans laquelle R¹ représente un atome d'hydrogène, un groupe alkyle présentant 1 à 10 atomes de carbone pouvant présenter un groupe hydroxyle, un groupe cycloalkyle présentant 5 à 10 atomes de carbone ou un groupe aralkyle présentant 7 à 10 atomes de carbone, et
un motif constitutif (1-4) présentant une structure représentée par la formule générale (1-f) ou une structure d'un sel d'addition d'acide de celui-ci : 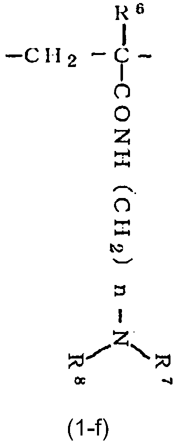
dans laquelle R⁶ représente un atome d'hydrogène ou un groupe méthyle, R⁷ et R⁸ représentent chacun indépendamment un groupe alkyle présentant 1 à 4 atomes de carbone et n est un entier de 2 à 4 ; et
au moins un type de motif constitutif anionique (2) sélectionné à partir du groupe constitué d'
un motif constitutif (2-1) présentant une structure représentée par la formule générale (II-a) : 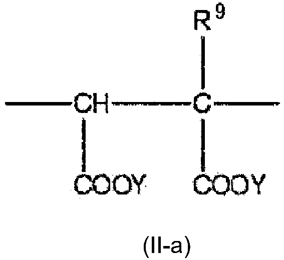
dans laquelle R⁹ est un atome d'hydrogène ou un groupe méthyle, Y représente un atome d'hydrogène, Na, K, NH₄, 1/2 Ca, 1/2 Mg, 1/2 Fe, 1/3 Al ou 1/3 Fe indépendamment pour chaque groupe carboxy à lier, et
un motif constitutif (2-4) présentant une structure représentée par la formule générale (II-d) : 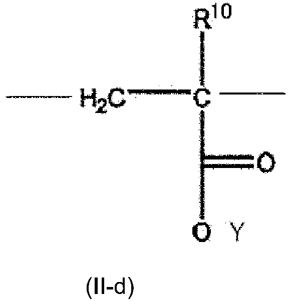
dans laquelle R¹⁰ est un atome d'hydrogène ou un groupe méthyle, Y représente un atome d'hydrogène, Na, K, NH₄, 1/2 Ca, 1/2 Mg, 1/2 Fe, 1/3 Al ou 1/3 Fe indépendamment pour chaque groupe carboxy à lier.

2. Utilisation d'un agent destiné à augmenter une température de fusion d'un acide nucléique double brin selon la revendication 1, dans laquelle le copolymère amphotère comprend en outre un motif constitutif non ionique (3).

3. Utilisation d'un agent destiné à augmenter la température de fusion selon la revendication 2, dans laquelle le motif constitutif non ionique (3) est un motif constitutif dérivé d'un monomère à base de méthacrylamide ou d'un monomère à base d'acrylamide.

4. Procédé destiné à détecter un mésappariement d'acides nucléiques entre un acide nucléique échantillon et un acide nucléique sonde, comprenant les étapes consistant à :
(1) mesurer des températures de fusion d'un acide nucléique double brin entre l'acide nucléique sonde et un acide nucléique présentant une séquence de bases complémentaire de l'acide nucléique sonde avec et sans l'addition d'un agent destiné à augmenter une température de fusion d'un acide nucléique double brin, et déterminer une valeur d'augmentation entre les températures de fusion de l'acide nucléique sonde et de l'acide nucléique présentant une séquence de bases complémentaire de l'acide nucléique sonde en raison de la présence du copolymère amphotère (ΔTm standard) ;
(2) mesurer des températures de fusion d'un acide nucléique double brin entre l'acide nucléique échantillon et l'acide nucléique sonde avec et sans l'addition d'un agent destiné à augmenter une température de fusion d'un acide nucléique double brin et déterminer une valeur d'augmentation entre les températures de fusion de l'acide nucléique double brin de l'acide nucléique échantillon et de l'acide nucléique sonde en raison de la présence du copolymère amphotère (ΔTm échantillon) ; et
(3) déterminer la présence ou l'absence de mésappariement d'acides nucléiques sur la base d'une valeur (ΔΔTm) obtenue en soustrayant le ΔTm échantillon de la ΔTm standard,
dans lequel l'agent destiné à augmenter une température de fusion d'un acide nucléique double brin comprend :
un copolymère amphotère, dans lequel le copolymère amphotère comprend :
au moins un type de motif constitutif cationique (1) sélectionné à partir du groupe constitué d'
un motif constitutif (1-1) présentant une structure représentée par la formule générale (I-a) ou la formule générale (I-b) ou une structure d'un sel d'addition d'acide de celui-ci : 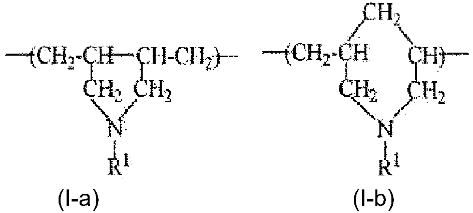
dans lequel R¹ représente un atome d'hydrogène, un groupe alkyle présentant 1 à 10 atomes de carbone pouvant présenter un groupe hydroxyle, un groupe cycloalkyle présentant 5 à 10 atomes de carbone ou un groupe aralkyle présentant 7 à 10 atomes de carbone, et
un motif constitutif (1-4) présentant une structure représentée par la formule générale (1-f) ou une structure d'un sel d'addition d'acide de celui-ci : 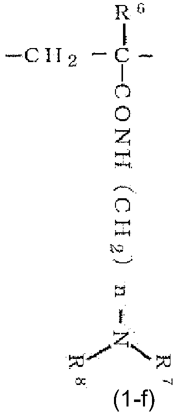
dans lequel R⁶ représente un atome d'hydrogène ou un groupe méthyle, R⁷ et R⁸ représentent chacun indépendamment un groupe alkyle présentant 1 à 4 atomes de carbone et n est un entier de 2 à 4 ; et
au moins un type de motif constitutif anionique (2) sélectionné à partir du groupe constitué d'
un motif constitutif (2-1) présentant une structure représentée par la formule générale (II-a) :] 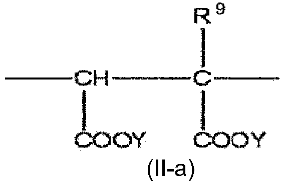
dans lequel R⁹ est un atome d'hydrogène ou un groupe méthyle, Y représente un atome d'hydrogène, Na, K, NH₄, 1/2 Ca, 1/2 Mg, 1/2 Fe, 1/3 Al ou 1/3 Fe indépendamment pour chaque groupe carboxy à lier, et
un motif constitutif (2-4) présentant une structure représentée par la formule générale (II-d) : 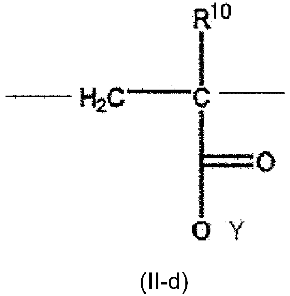
dans lequel R¹⁰ est un atome d'hydrogène ou un groupe méthyle, Y représente un atome d'hydrogène, Na, K, NH₄, 1/2 Ca, 1/2 Mg, 1/2 Fe, 1/3 Al ou 1/3 Fe indépendamment pour chaque groupe carboxy à lier.

5. Procédé destiné à détecter un mésappariement d'acides nucléiques selon la revendication 4, dans lequel il est déterminé que le mésappariement d'acides nucléiques est présent lorsque la ΔΔTm est de 5,0 °C ou plus.

6. Procédé destiné à détecter un mésappariement d'acides nucléiques selon la revendication 4 ou la revendication 5, dans lequel le copolymère amphotère comprend en outre un motif constitutif non ionique (3).

7. Procédé destiné à détecter un mésappariement d'acides nucléiques selon la revendication 6, dans lequel le motif constitutif non ionique (3) est un motif constitutif dérivé d'un monomère à base de méthacrylamide ou d'un monomère à base d'acrylamide.

8. Kit destiné à :
(i) détecter un mésappariement d'acides nucléiques entre un acide nucléique échantillon et un acide nucléique sonde, comprenant l'acide nucléique sonde et un acide nucléique présentant une séquence de bases complémentaire de l'acide nucléique sonde ; ou
(ii) détecter une mutation entre un acide nucléique échantillon et un acide nucléique standard, comprenant l'acide nucléique standard et un acide nucléique présentant une séquence de bases complémentaire de l'acide nucléique standard ;
dans lequel le kit comprend
un agent destiné à augmenter une température de fusion d'un acide nucléique double brin,
dans lequel l'agent destiné à augmenter une température de fusion d'un acide nucléique double brin comprend :
un copolymère amphotère, dans lequel le copolymère amphotère comprend :
au moins un type de motif constitutif cationique (1) sélectionné à partir du groupe constitué d'
un motif constitutif (1-1) présentant une structure représentée par la formule générale (I-a) ou la formule générale (I-b) ou une structure d'un sel d'addition d'acide de celui-ci : 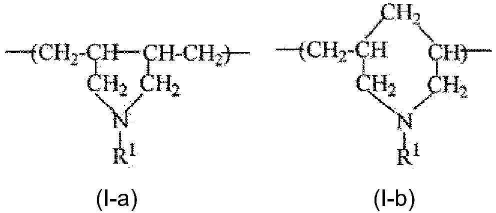
dans lequel R¹ représente un atome d'hydrogène, un groupe alkyle présentant 1 à 10 atomes de carbone pouvant présenter un groupe hydroxyle, un groupe cycloalkyle présentant 5 à 10 atomes de carbone ou un groupe aralkyle présentant 7 à 10 atomes de carbone, et
un motif constitutif (1-4) présentant une structure représentée par la formule générale (1-f) ou une structure d'un sel d'addition d'acide de celui-ci : 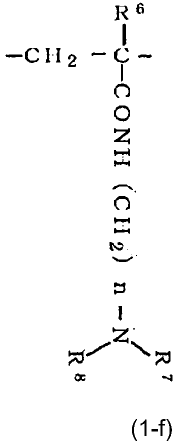
dans lequel R⁶ représente un atome d'hydrogène ou un groupe méthyle, R⁷ et R⁸ représentent chacun indépendamment un groupe alkyle présentant 1 à 4 atomes de carbone et n est un entier de 2 à 4 ; et
au moins un type de motif constitutif anionique (2) sélectionné à partir du groupe constitué d'
un motif constitutif (2-1) présentant une structure représentée par la formule générale (II-a) : 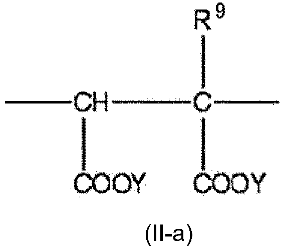
dans lequel R⁹ est un hydrogène ou un groupe méthyle, Y représente un hydrogène, Na, K, NH₄, 1/2 Ca, 1/2 Mg, 1/2 Fe, 1/3 Al ou 1/3 Fe indépendamment pour chaque groupe carboxy à lier, et
un motif constitutif (2-4) présentant une structure représentée par la formule générale (II-d) : 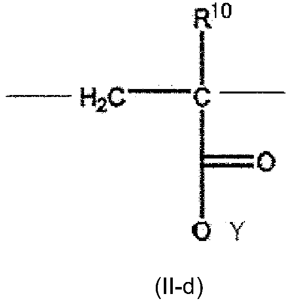
dans lequel R¹⁰ est un atome d'hydrogène ou un groupe méthyle, Y représente un atome d'hydrogène, Na, K, NH₄, 1/2 Ca, 1/2 Mg, 1/2 Fe, 1/3 Al ou 1/3 Fe indépendamment pour chaque groupe carboxy à lier.

9. Kit destiné à détecter un mésappariement d'acides nucléiques selon la revendication 8, dans lequel le copolymère amphotère comprend en outre un motif constitutif non ionique (3).

10. Kit destiné à détecter un mésappariement d'acides nucléiques selon la revendication 9, dans lequel le motif constitutif non ionique (3) est un motif constitutif dérivé d'un monomère à base de méthacrylamide ou un monomère à base d'acrylamide.

11. Procédé destiné à la détection d'une mutation par rapport à un acide nucléique standard dans un acide nucléique échantillon, comprenant les étapes consistant à :
(1) mesurer des températures de fusion d'un acide nucléique double brin entre l'acide nucléique standard et un acide nucléique présentant une séquence de bases complémentaire de l'acide nucléique standard avec et sans l'addition d'un agent destiné à augmenter une température de fusion d'un acide nucléique double brin, et déterminer une valeur d'augmentation (ΔTm standard) entre les températures de fusion de l'acide nucléique double brin entre l'acide nucléique standard et l'acide nucléique présentant la séquence de bases complémentaire de l'acide nucléique standard en raison de la présence du copolymère amphotère ;
(2) mesurer des températures de fusion d'un acide nucléique double brin de l'acide nucléique échantillon et de l'acide nucléique présentant une séquence de bases complémentaire de l'acide nucléique standard avec et sans l'addition d'un agent destiné à augmenter une température de fusion d'un acide nucléique double brin, et déterminer une valeur d'augmentation (ΔTm échantillon) entre les températures de fusion de l'acide nucléique double brin entre l'acide nucléique échantillon et l'acide nucléique présentant la séquence de bases complémentaire de l'acide nucléique standard en raison de la présence du copolymère amphotère ; et
(3) déterminer la présence ou l'absence de mutation sur la base d'une valeur (ΔΔTm) obtenue en soustrayant l'ATM échantillon de la ΔTm standard,
dans lequel l'agent destiné à augmenter une température de fusion d'un acide nucléique double brin comprend :
un copolymère amphotère, dans lequel le copolymère amphotère comprend :
au moins un type de motif constitutif cationique (1) sélectionné à partir du groupe constitué d'
un motif constitutif (1-1) présentant une structure représentée par la formule générale (I-a) ou la formule générale (I-b) ou une structure d'un sel d'addition d'acide de celui-ci : 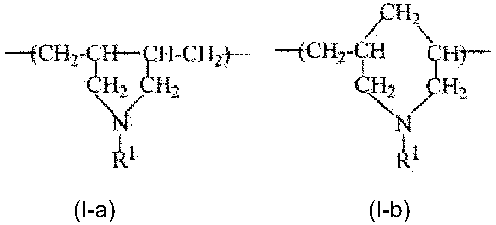
dans lequel R¹ représente un atome d'hydrogène, un groupe alkyle présentant 1 à 10 atomes de carbone qui peut présenter un groupe hydroxyle, un groupe cycloalkyle présentant 5 à 10 atomes de carbone ou un groupe aralkyle présentant 7 à 10 atomes de carbone, et
un motif constitutif (1-4) présentant une structure représentée par la formule générale (1-f) ou une structure d'un sel d'addition d'acide de celui-ci : 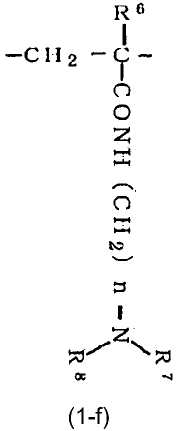
dans lequel R⁶ représente un atome d'hydrogène ou un groupe méthyle, R⁷ et R⁸ représentent chacun indépendamment un groupe alkyle présentant 1 à 4 atomes de carbone et n est un entier de 2 à 4 ; et
au moins un type de motif constitutif anionique (2) sélectionné à partir du groupe constitué d'
un motif constitutif (2-1) présentant une structure représentée par la formule générale (II-a) : 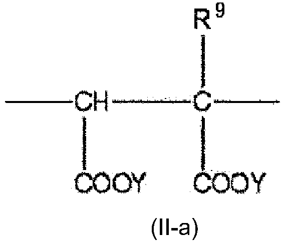
dans lequel R⁹ est un atome d'hydrogène ou un groupe méthyle, Y représente un atome d'hydrogène, Na, K, NH₄, 1/2 Ca, 1/2 Mg, 1/2 Fe, 1/3 Al ou 1/3 Fe indépendamment pour chaque groupe carboxy à lier, et
un motif constitutif (2-4) présentant une structure représentée par la formule générale (II-d) : 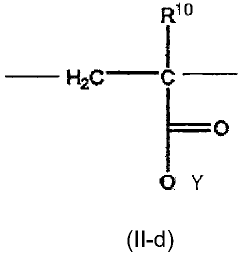
dans lequel R¹⁰ est un atome d'hydrogène ou un groupe méthyle, Y représente un atome d'hydrogène, Na, K, NH₄, 1/2 Ca, 1/2 Mg, 1/2 Fe, 1/3 Al ou 1/3 Fe indépendamment pour chaque groupe carboxy à lier.

12. Procédé destiné à la détection d'une mutation selon la revendication 11, dans lequel il est déterminé qu'une mutation est présente lorsque la ΔΔTm est de 5,0° C ou plus.

13. Procédé destiné à la détection d'une mutation selon la revendication 11 ou la revendication 12, dans lequel le copolymère amphotère comprend en outre un motif constitutif non ionique (3), de préférence dans lequel le motif constitutif non ionique (3) est un motif constitutif dérivé d'un monomère à base de méthacrylamide ou d'un monomère à base d'acrylamide.
